# EUROPÄISCHE PATENTSCHRIFT

(11) **EP 0 441 951 B1**
(45) Veröffentlichungstag und Bekanntmachung des Hinweises auf die Patenterteilung: **10.07.1996**
(21) Anmeldenummer: 90914154.1
(22) Anmeldetag: 30.08.1990
(51) Int. Cl.: C07C 22/08, C09K 19/30, G02F 1/13, C07C 25/18, C07C 22/04, C07C 255/50, C07C 43/225

(54) **HALOGENIERTE BENZOLDERIVATE UND FLÜSSIGKRISTALLINES MEDIUM**
HALOGENATED BENZENE DERIVATIVES AND LIQUID CRYSTALLINE MEDIUM
DERIVES HALOGENES DE BENZENE ET MILIEU MESOMORPHE

(30) Priorität: 30.08.1989 DE 3928656; 30.08.1989 DE 3928657; 28.03.1990 DE 4009928; 22.01.1990 GB 9001408
(43) Veröffentlichungstag der Anmeldung: 21.08.1991
(73) Patentinhaber: MERCK PATENT GmbH, D-64271 Darmstadt (DE)
(72) Erfinder: HITTICH, Reinhard, D-6101 Modautal 1 (DE); POETSCH, Eike, D-6109 Mühltal 9 (DE); REIFFENRATH, Volker, D-6101 Rossdorf (DE); RIEGER, B. Wacoe, Tamagawagakuen 2834, Ootadaira, Kanagawa, Pref. 227 (JP); WÄCHTLER, Andreas, D-6103 Griesheim (DE); COATES, David, Dorset BH21 3SW (GB); PLACH, Herbert, D-6100 Darmstadt (DE)
(86) Internationale Anmeldenummer: EP9001437
(87) Internationale Veröffentlichungsnummer: WO9103445

(56) Entgegenhaltungen:
- EP-A- 0 102 047
- EP-A- 0 144 648
- EP-A- 0 149 208
- EP-A- 0 205 998
- EP-A- 0 256 636
- EP-A- 0 272 580
- EP-A- 0 280 902
- EP-A- 0 291 949
- EP-A- 0 316 715
- DE-A- 3 223 637
- DE-A- 3 233 641
- DE-A- 3 921 836
- JP-A- 6 092 228
- Chemical Abstract, Band 110, 1989, (Columbus, Ohio, US),siehe Seite 760

## Beschreibung

Die Erfindung betrifft neue halogenierte Benzolderivate der Formel I 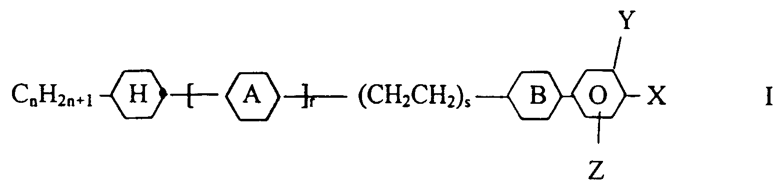 worin n 1 bis 10, die Ringe A und B jeweils trans-1,4-Cyclohexylen, oder Ring B auch 1,4-Phenylen oder 3-Fluor-1,4-phenylen, X F, Cl, -OCF₃ oder -OCHF₂, Y und Z jeweils unabhängig voneinander H oder F bedeuten, r = 1 oder im Falle s = 1, B = trans-1,4-Cyclohexylen und gleichzeitig X = -OCF₃ oder -OCHF₂ auch r = 0, und s = 1 oder im Falle B = 1,4-Phenylen oder 3-Fluor- 1,4-phenylen auch s = 0 bedeutet,
mit der Maßgabe, daß
im Falle r = 1, A = trans-1,4-Cyclohexylen und gleichzeitig X = F B 3-Fluor-1,4-phenylen oder trans-1,4-Cyclohexylen und/oder Y und Z jeweils Fluor bedeuten, wobei Verbindungen der Formel 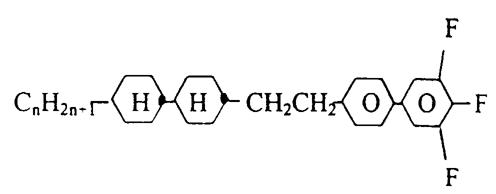 mit n = 1 - 10 ausgeschlossen sind.

Die Erfindung betrifft ferner flüssigkristalline Medien für elektrooptische Anzeigen mit einem oder mehreren halogenierten Benzolderivaten der Formel I 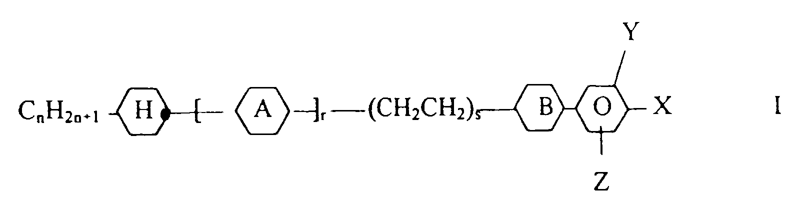 worin n 1 bis 10, die Ringe A und B jeweils trans-1,4-Cyclohexylen, oder Ring B auch 1,4-Phenylen oder 3-Fluor-1,4-phenylen, X F, Cl, -OCF₃ oder -OCHF₂, Y und Z jeweils unabhängig voneinander H oder F bedeuten, r = 1 oder im Falle s = 1, B = trans-1,4-Cyclohexylen und gleichzeitig X = -OCF₃ oder -OCHF₂ auch r = 0, und s = 1 oder im Falle B = 1,4-Phenylen oder 3-Fluor-1,4-phenylen auch s = 0 bedeutet,
oder der folgenden Formel 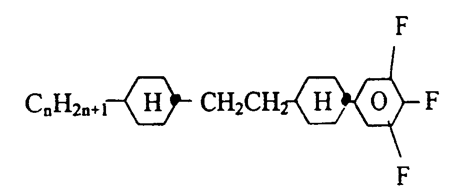 (n = 2, 3, 5)
dadurch gekennzeichnet, daß es als weiteren Bestandteil eine Verbindung der Formel 1 enthält

R'-L-E-R" I

worin L und E, die gleich oder verscheiden sein können, jeweils unabhängig voneinander einen bivalenten Rest aus der aus -Phe-, -Phe-Phe-, -Phe-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc-trans-1,4-Cyclohexylen oder 1,4-Cyclo-hexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten,
R' und R" entweder
   jeweils unabhängig voneinander Alkyl, Alkenyl, Alkoxy, Alkoxyalkyl, Alkenyloxy oder Alkanoyloxy mit bis zu 8 Kohlenstoffatomen, oder
R" -F, -Cl, -NCS oder -(O)ᵢCH₃₋₍ₖ₊₁₎FₖClₗ, wobei i 0 oder 1 und k + 1 1, 2 oder 3 sind und R' Alkyl, Alkenyl, Alkoxy oder Alkoxyalkyl mit bis zu 8 Kohlenstoffatomen, oder
R" -CN und R' Alkyl, Alkoxy oder Alkenyl mit bis zu 8 Kohlenstoffatomen bedeutet.

Aus den EP-OS 0 205 998 und 0 291 949 sind Flüssigkristalle der Formel A1 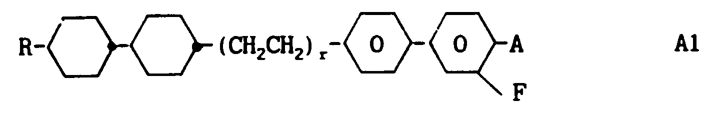 bekannt, worin A F oder CN und R Alkyl mit 1 bis 7 C-Atomen bedeutet.

Die Verbindungen mit A = F zeichnen sich jedoch nur durch eine moderate dielektrische Anisotropie aus, die in Mischungen oft zu relativ hohen Schwellenspannungen führt.

Darüber hinaus ist die Mischungbarkeit der Verbindungen A1 mit anderen Flüssigkristallverbindungen in manchen Fällen verbesserungsbedürftig.

Weitere, ähnliche Nitrilverbindungen sind bekannt:

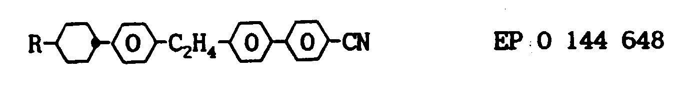

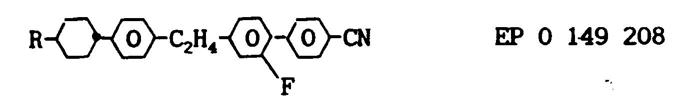

Aus der EP-OS 0 280 902 sind Flüssigkristalle der Formel A2 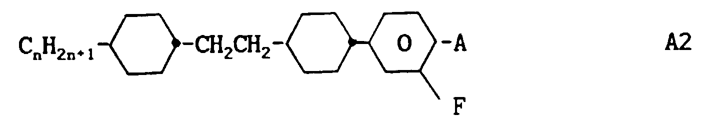 bekannt, worin A H, F, Cl, Br oder CN bedeutet.

Aus der EP-OS 0 272 580 sind Flüssigkristalle der Formel B bekannt 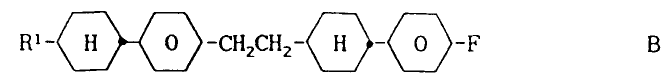 worin R¹ C₂H₅ oder C₄H₇ bedeutet.

Die Verbindungen A2 werden jedoch entweder nicht den hohen Anforderungen an den elektrischen Widerstand gerecht (A = CN), wie sie beispielsweise für Anzeigen mit aktiver Matrix gefordert werden, oder zeichnen sich nur durch eine moderate dielektrische Anisotropie aus, die in Mischungen oft zu relativ hohen Schwellenspannungen führt (A = H, F, Cl oder Br). Die Verbindungen B hingegen zeichnen sich durch recht hohe Schmelzpunkte und relativ ungünstige Mischbarkeit mit anderen Flüssigkristallverbindungen aus. Darüberhinaus ist deren dielektrische Anisotropie recht gering.

Weitere ähnliche Verbindungen sind in den folgenden Dokumenten beschrieben:
EP-A- 0 256 636, JP-A- 6092228, DE-A- 32 23 637 und EP-A- 0 387 032

Die Verbindungen der Formel I können wie ähnliche, z.B. aus der DE-OS 33 17 597 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung, Induzierung von smektischen Phasen bei tiefen Temperaturen und/oder zu geringe Löslichkeit bei tiefen Temperaturen.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit breiten nematischen Bereichen, hohem Klärpunkt, hervorragender Nematogenität bis zu tiefen Temperaturen, hervorragender chemischer Stabilität, ausgeprägtem ε _{┴} bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere bzw. in anderen Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben n, r, s, A, B, X, Y und Z die ange- gebene Bedeutung, sofern nicht ausdrücklich etwas anderes vermerkt ist.

In den Verbindungen der Formel I sind die Alkylgruppen CₙH₂ₙ₊₁ vorzugsweise geradkettig. Dementsprechend bedeutet CₙH₂ₙ₊₁ Methyl, Ethyl, n-Propyl, n-Butyl, n-Pentyl, n-Hexyl oder n-Heptyl. n ist vorzugsweise 1 bis 7 und insbesondere bevorzugt 2, 3, 4 oder 5.

Verbindungen der Formel I mit verzweigten Alkylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl), 2-Methylbutyl, Isopentyl (= 3-Methylbutyl), 2-Methylpentyl, 3-Methylpentyl oder 2-Heptyl (= 1-Methylhexyl).

Der Rest 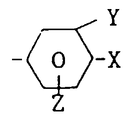 ist vorzugsweise

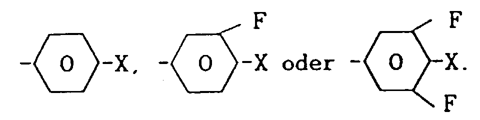

Besonders bevorzugt sind die Verbindungen der folgenden Teilformeln: 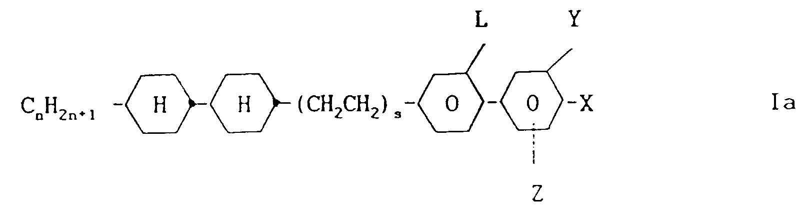 worin n 1 bis 7, s 0 oder 1, X F, Cl, -OCF₃ oder -OCHF₂ und Y, L und Z jeweils unabhängig voneinander H oder F bedeuten, mit der Maßgabe, daß im Falle X = F L = F, L = Y = F oder Y = Z = F. 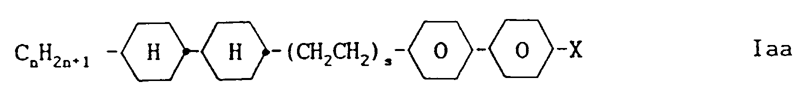 worin X Cl, -OCF₃ oder -OCHF₂ ist. 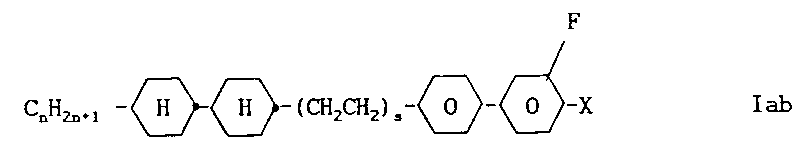 worin X Cl, -OCF₃ oder -OCHF₂ ist. 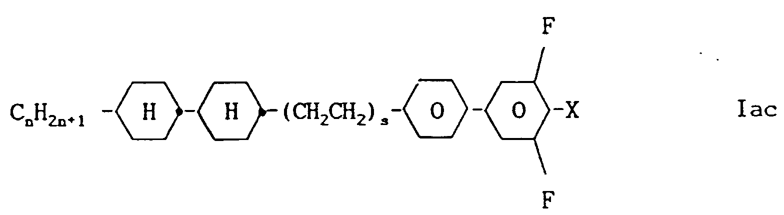 worin X F, Cl, -OCF₃ oder -OCHF₂ ist. 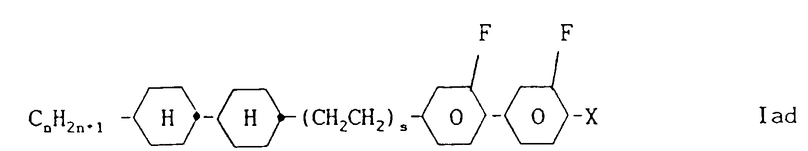 worin X F, Cl, -OCF₃ oder -OCHF₂ ist. 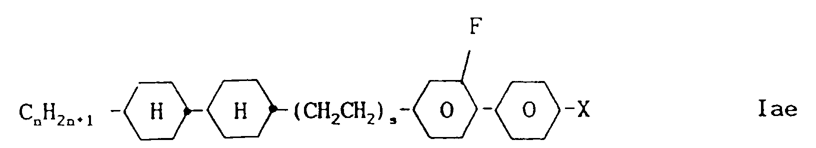 worin X F, Cl, -OCF₃ oder -OCHF₂ ist. 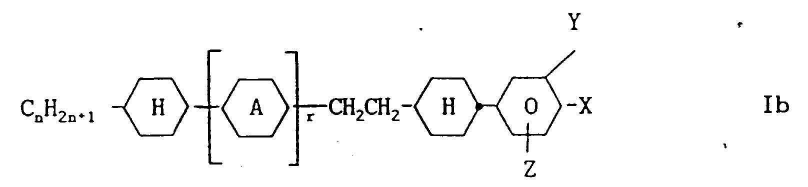 worin n 1 bis 7, r 0 oder 1, Ring A trans-1,4-Cyclohexylen X F, Cl, -OCF₃ oder -OCHF₂ und Y und Z jeweils unabhängig voneinander H oder F bedeuten, 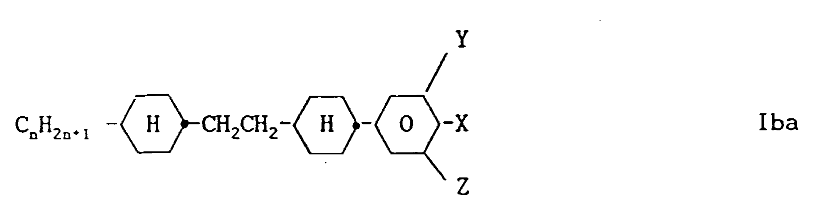 worin X -OCF₃ oder -OCHF₂ bedeutet und Y und Z jeweils unabhängig voneinander H oder F bedeuten. 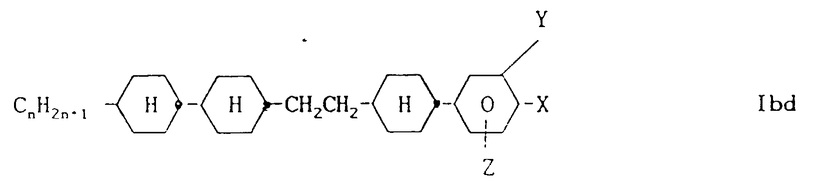 worin X, Y und Z die in Anspruch 8 angegebene Bedeutung haben, vorzugsweise
(a) X = F, Cl, -OCF₃ oder -OCHF₂ und Y = Z = H,
(b) X = F, Cl, -OCF₃ oder -OCHF₂, insbesondere F oder Cl, Y = F und Z = H.
X = F, Cl, -OCF₃ oder -OCHF_{2.} Y = F und Z = H (X = F oder Cl ist besonders bevorzugt).

Besonders bevorzugt sind ferner die Verbindungen der folgenden Teilformeln: 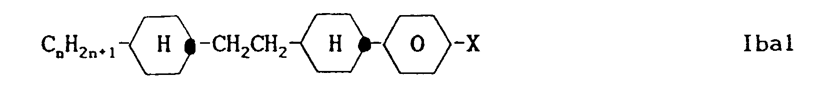 worin X -OCF₃ oder -OCHF₂ bedeutet, 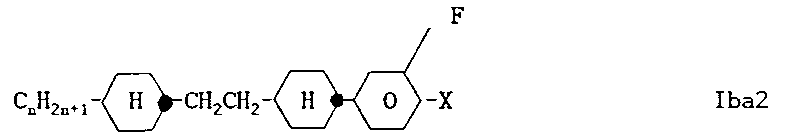 worin X -OCF₃ oder -OCHF₂ bedeutet, 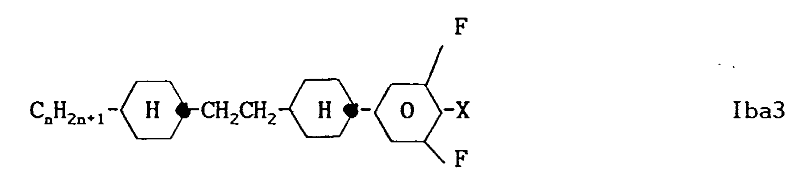 worin X F, Cl, -OCF₃ oder -OCHF₂ bedeutet.

Die Verbindungen der Formel I werden im übrigen nach an sich bekannten Methoden hergestellt, wie sie in der Literatur (z.B. in den Standardwerken wie Houben-Weyl, Methoden der Organischen' Chemie, Georg-Thieme-Verlag, Stuttgart) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I, worin r = 1 und A trans-1,4-Cyclohexylen bedeutet, können beispielsweise durch Umsetzung eines Aldehyds der Formel II 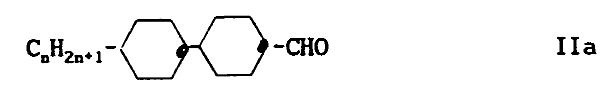 mit einem Phosphoniumsalz, z.B. einem entsprechenden p-(subst. Phenyl)-benzylbromid nach Wittig und katalytischer Hydrierung z.B. an Pd/C der erhaltenen Ethenderivate erhalten werden.

Zur Synthese der Benzylbromide geeignete Vorstufen sind beispielsweise nach folgendem Syntheseschema erhältlich:

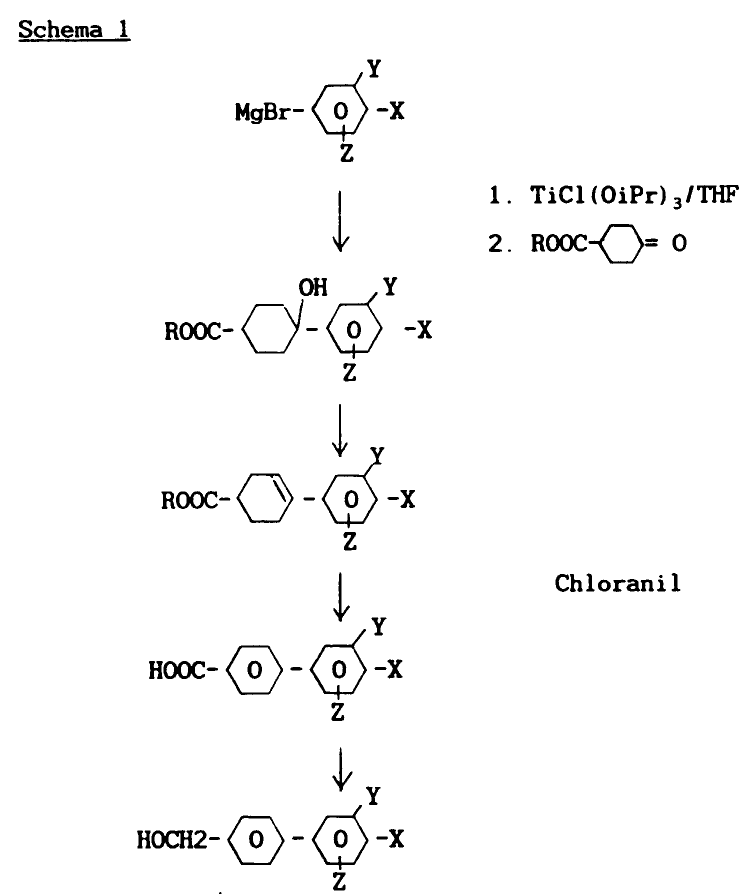

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Dehydrierung und Reduktion zum Benzylalkohol erhält man die geeignete Vorstufe.

Die als Ausgangsstoffe verwendete Brombenzolderivate sind zum Teil bekannt, zum Teil können sie ohne Schwierigkeiten nach Standardverfahren der organischen Chemie aus literatur-bedingten Verbindungen hergestellt werden. Beispielsweise sind die OCF₃- oder OCHF₂-Verbindungen nach bekannten Verfahren aus den entsprechenden Phenolen erhältlich. Verbindungen der Formel 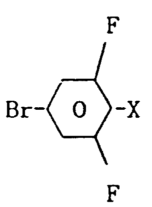 oder auch entsprechende monofluorierte Verbindungen sind beispielsweise aus den bekannten Vorstufen mit X = H durch Lithiierung bei tiefen Temperaturen und anschließende Umsetzung mit einem geeigneten Elektrophil erhältlich.

Die Verbindungen der Formel I mit B = 3-Fluor-1,4-phenylen erhält man in völliger Analogie durch Einsatz von 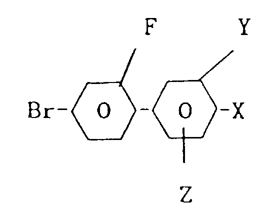 anstelle der Brombenzolderivate. Die Brombiphenyle können in an sich bekannter Weise durch edelmetallkatalysierte Kreuzkopplungsreaktionen hergestellt werden (E. Poetsch, Kontakte (Darmstadt) 1988 (2) p. 15).

Eine Variante ist beispielhaft im folgenden angegeben:

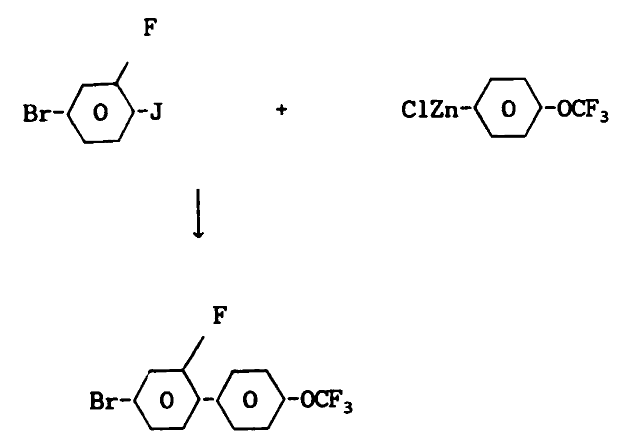

Weitere Synthesevarianten sind dem Fachmann bekannt. Bevorzugte Varianten sind den folgenden Schemata zu entnehmen. Alle Ausgangsmaterialien sind entweder bekannt oder können in Analogie zu bekannten Verbindungen hergestellt werden.

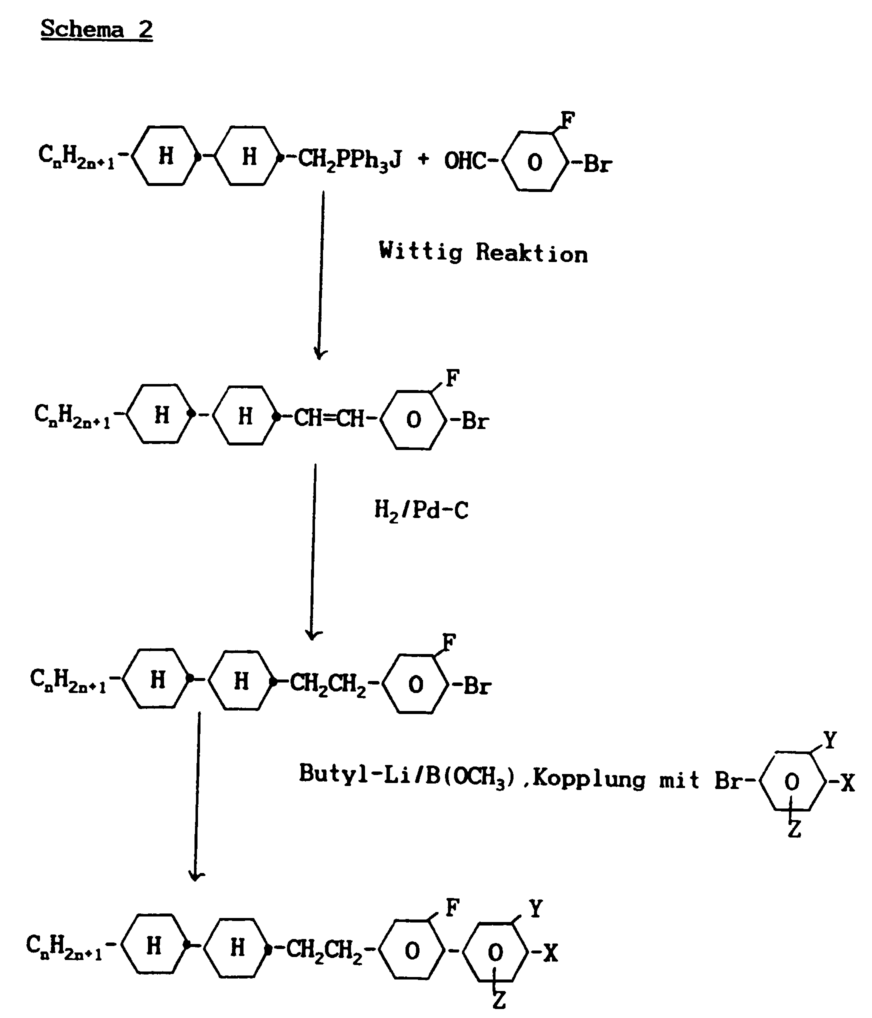

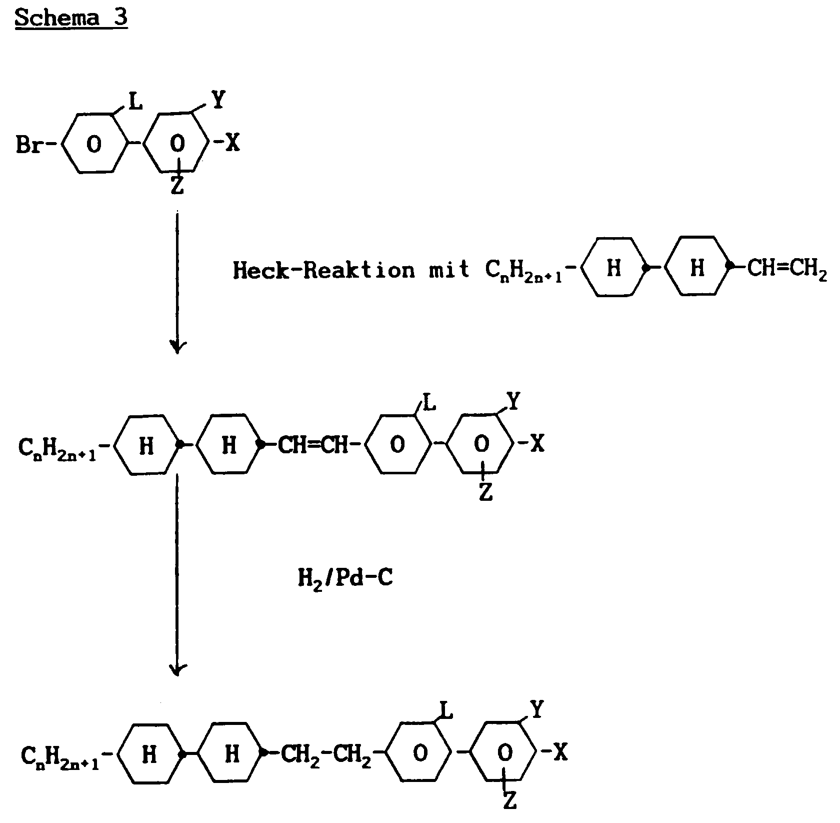

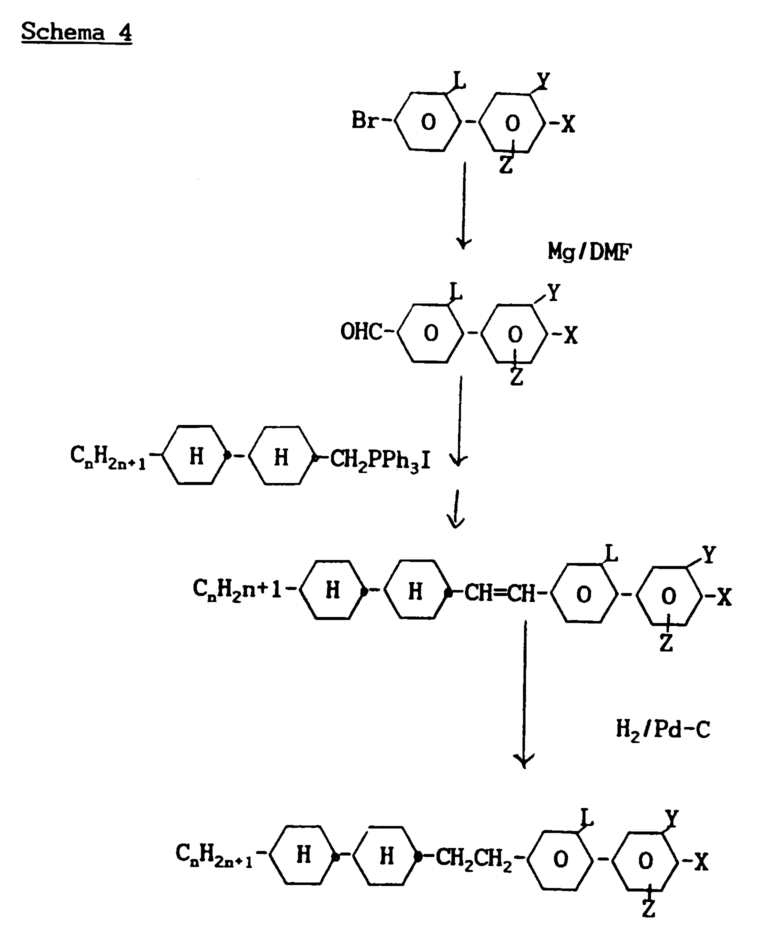

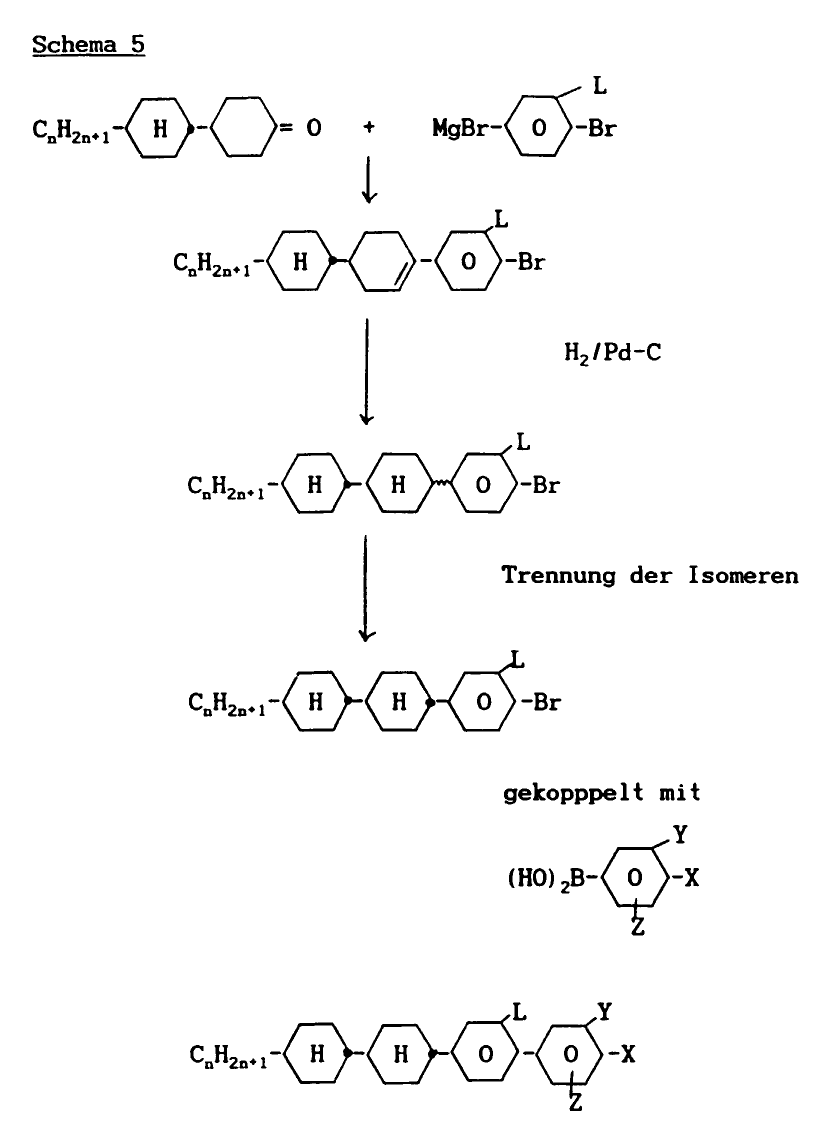

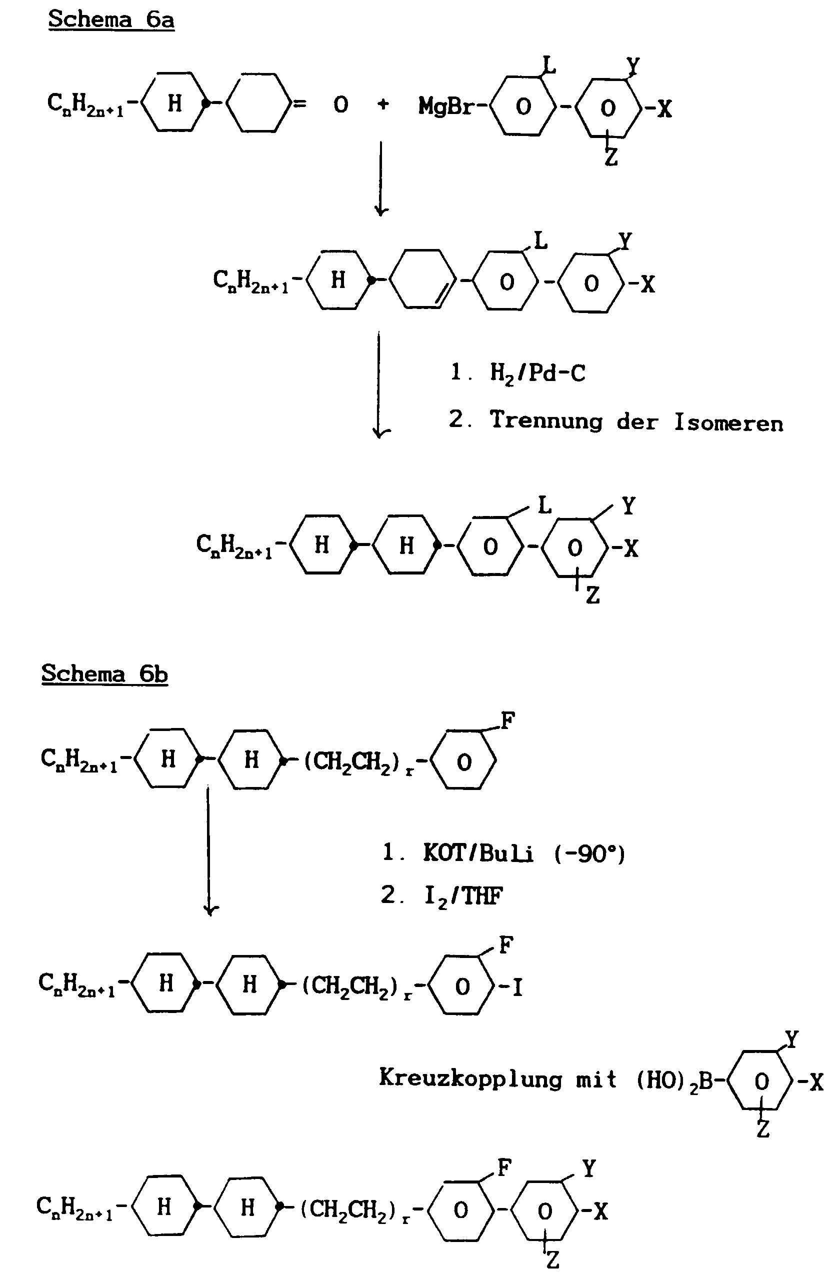

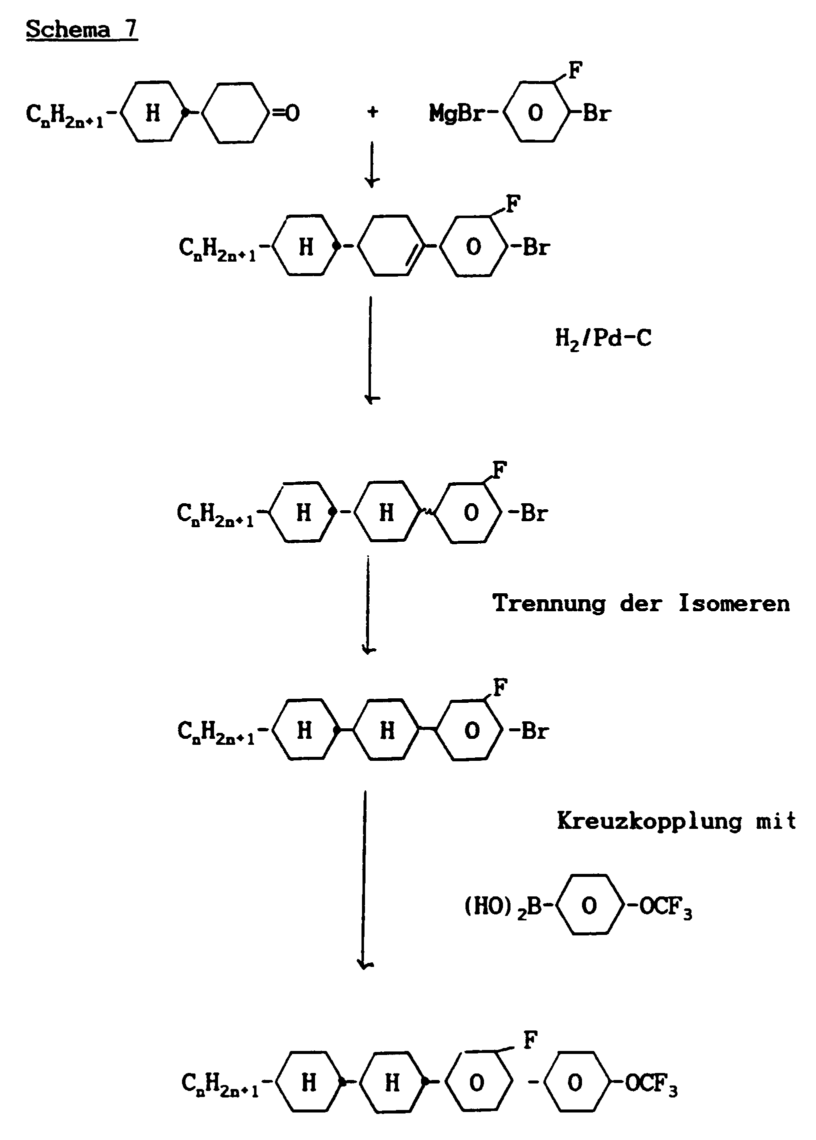

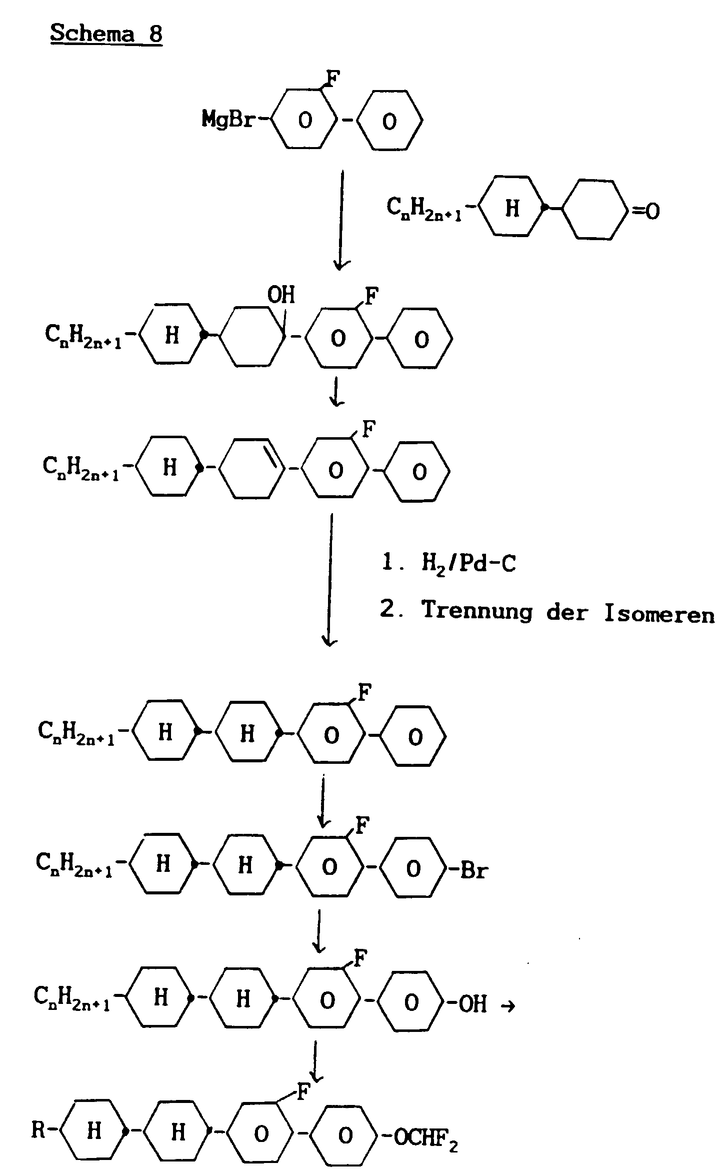

Die Verbindungen der Formel Ib können beispielsweise durch Umsetzung eines Aldehyds der Formel IIb 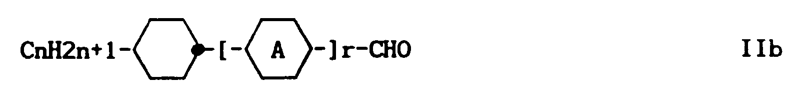 mit dem Phosphoniumsalz eines entsprechenden trans-4-(sub-st.Phenyl)-cyclohexylmethylbromids nach Wittig und katalytischer Hydrierung, z.B. an Pd/C der erhaltenen Ethenderivate, erhalten werden.

Zur Synthese der Cyclohexylmethylbromide geeignete Vorstufen sind beispielsweise nach folgendem Synthesechema erhältlich:

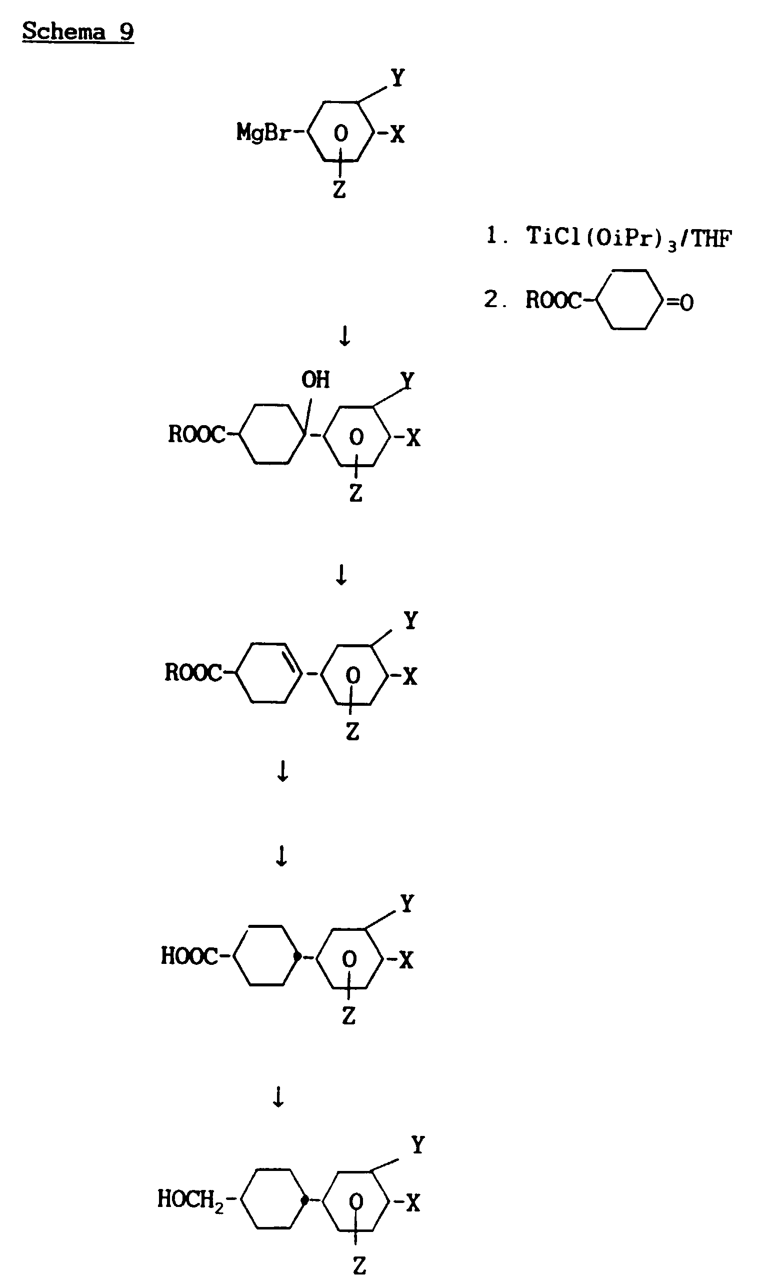

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung, Isomerisierung und Reduktion zum Cyclohexylmethylalkohol erhält man die geeignete Vorstufe.

Weitere Synthesevarianten sind dem Fachmann bekannt. Bevorzugte Varianten sind den folgenden Schemata zu entnehmen. Alle Ausgangsmaterialien sind entweder bekannt oder können in Analogie zu bekannten Verbindungen hergestellt werden. Das Cyclohexylessigsäurechlorid in Schema 11 läßt sich durch übliche Homologisierung der entsprechenden Cyclohexancarbonsäure aus Schema 9 herstellen.

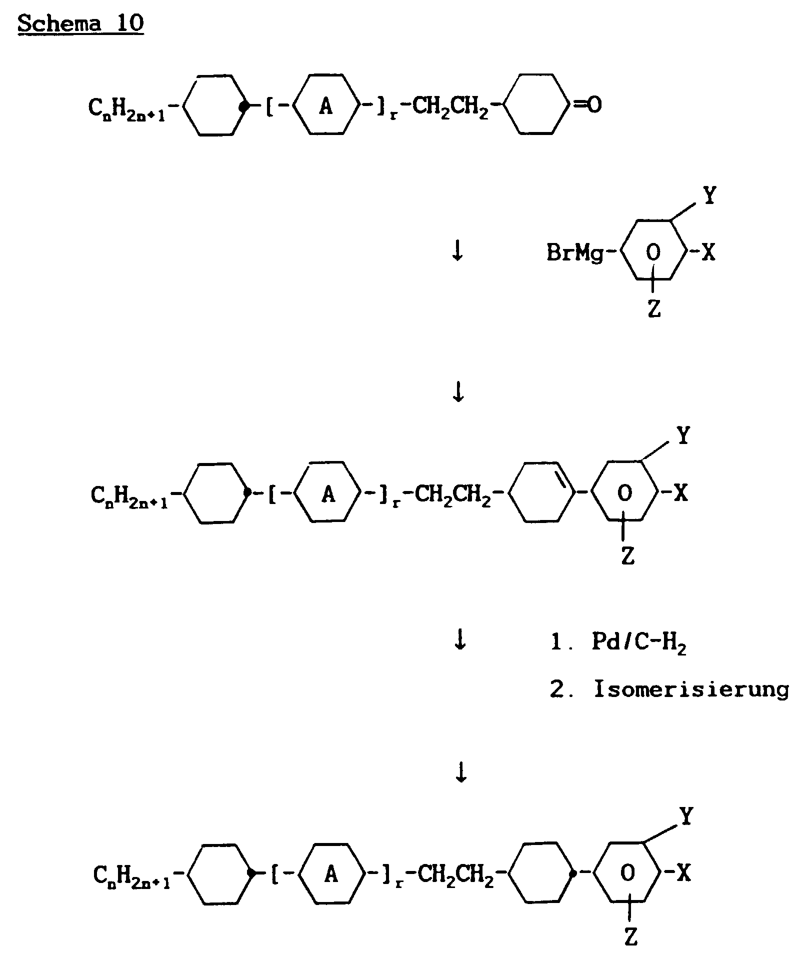

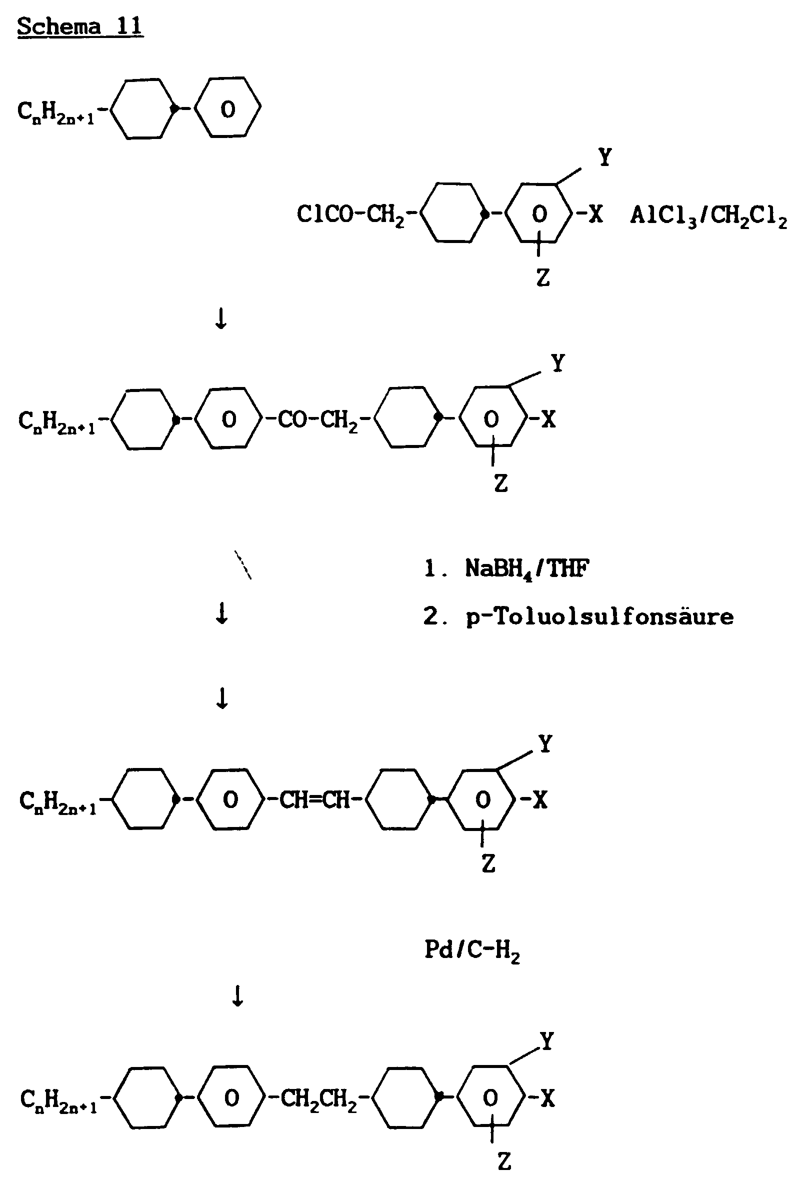

Weitere Syntheseschemata für besonders bevorzugte Verbindungen:

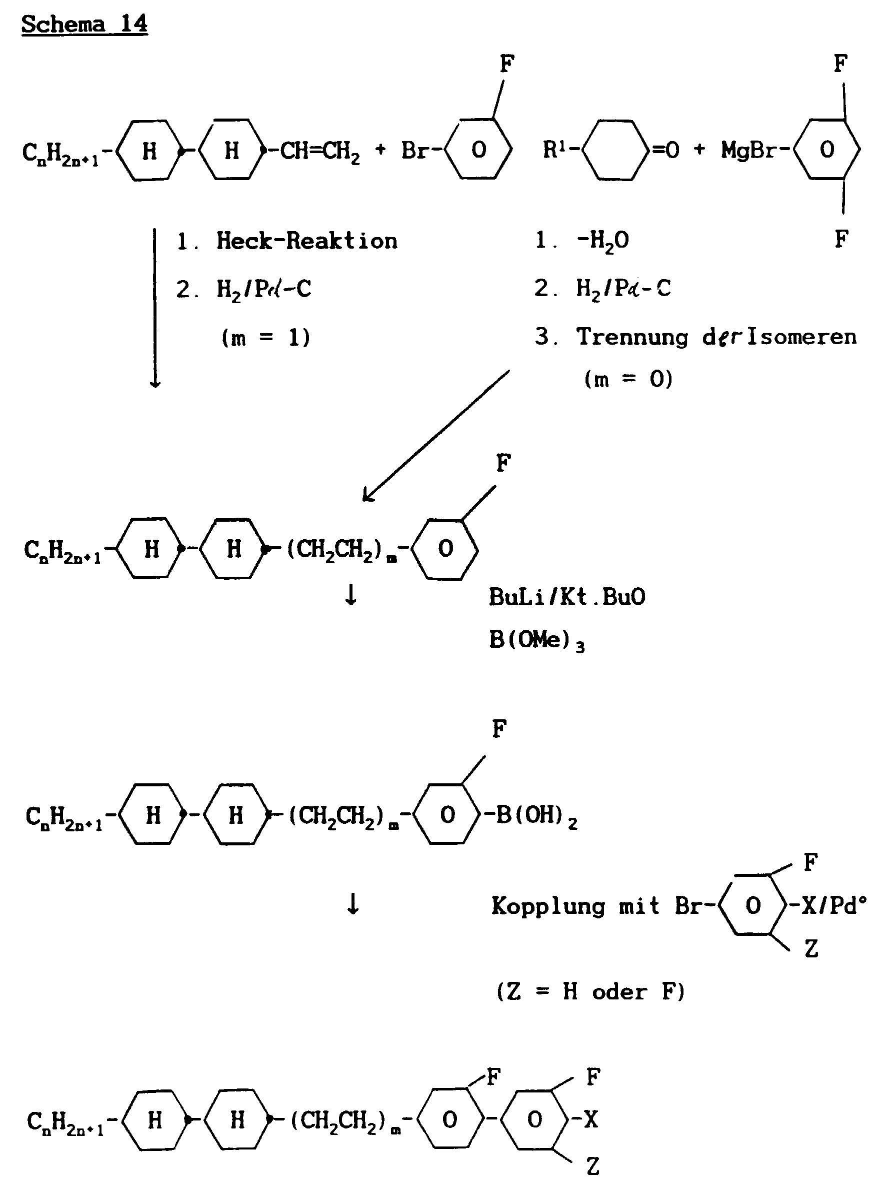

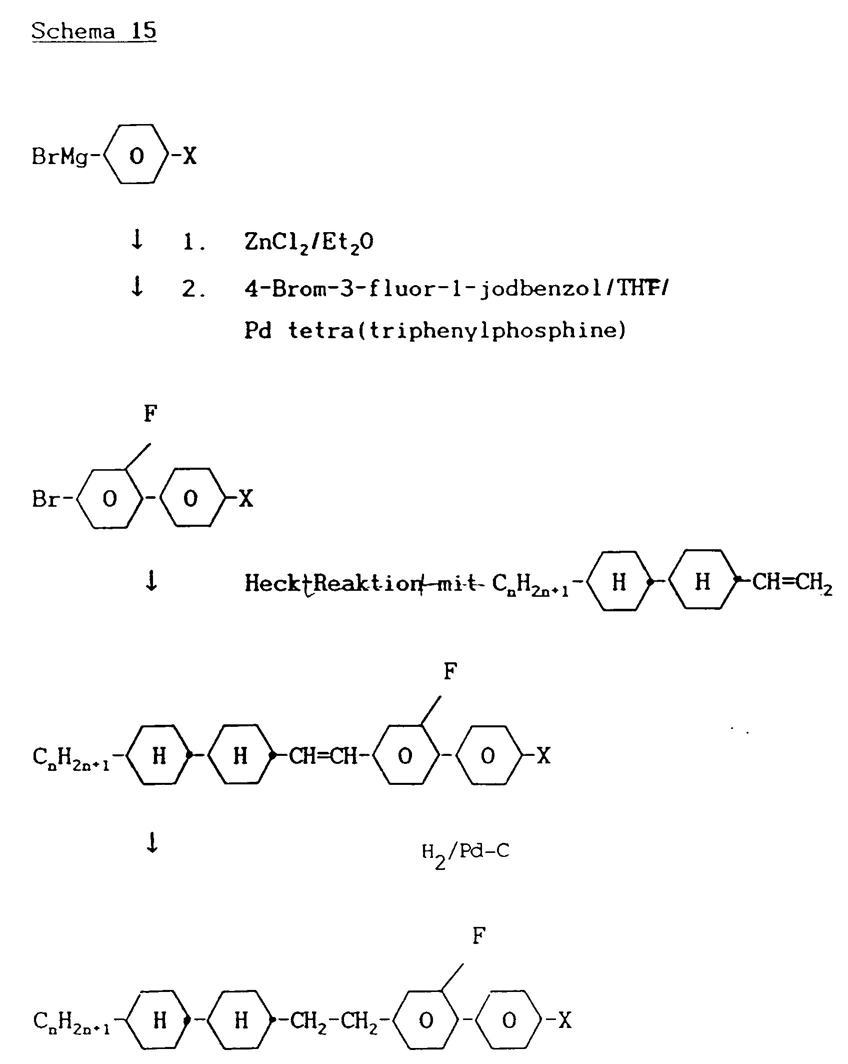

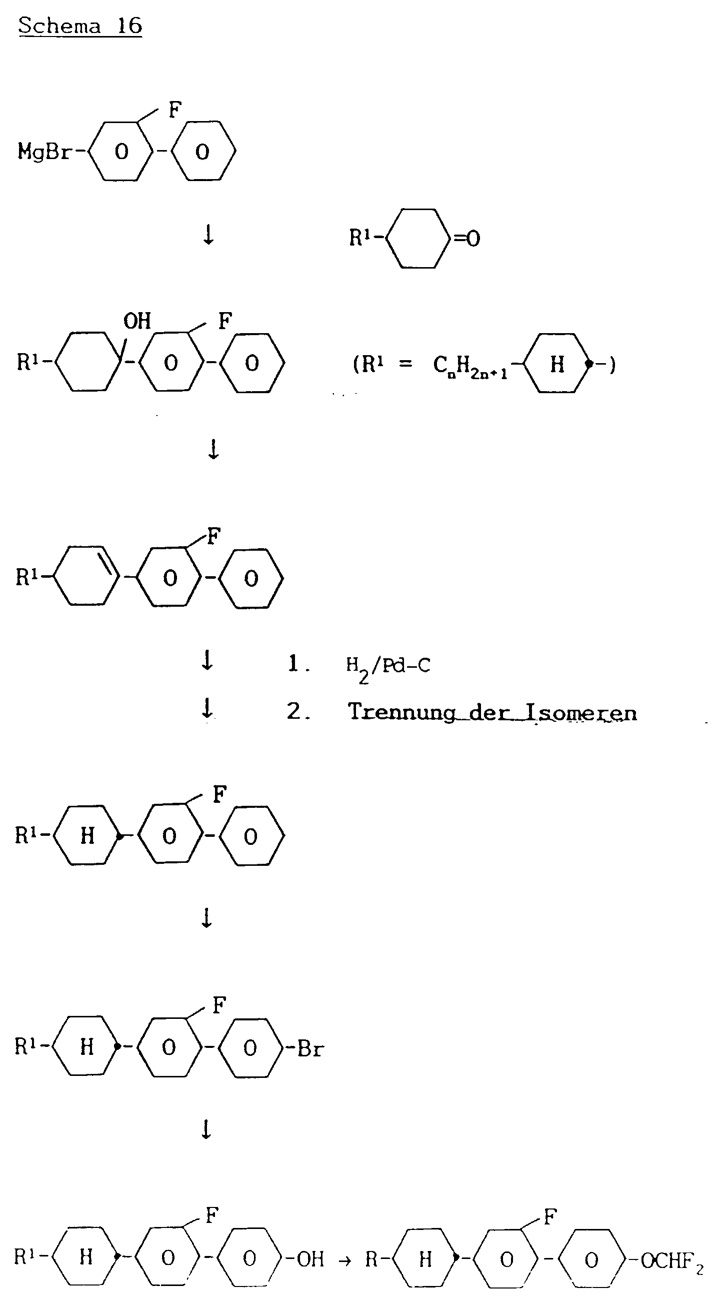

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Biscyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C=C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl) -ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln la, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋₍ₖ₊₁₎FₖCl₁, wobei i 0 oder 1 und k+1 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln la-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich.Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nT | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nOmFF | CₙH₂ₙ₊₁ | OCₘH₂ₘ₊₁ | F | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nNF | CₙH₂ₙ₊₁ | CN | F | H |
| nAm | CₙH₂ₙ₊₁ | COOCₘH₂ₘ₊₁ | H | H |

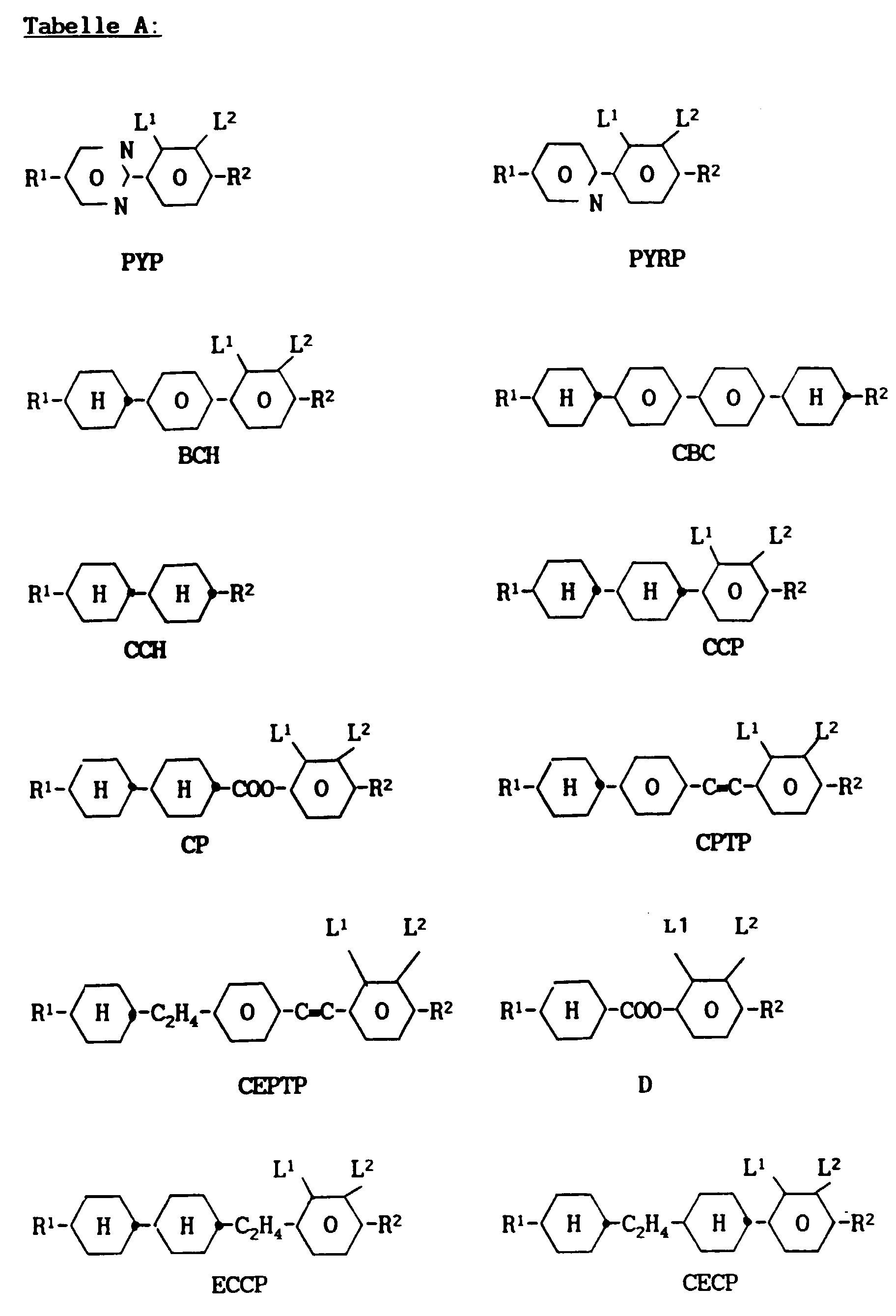

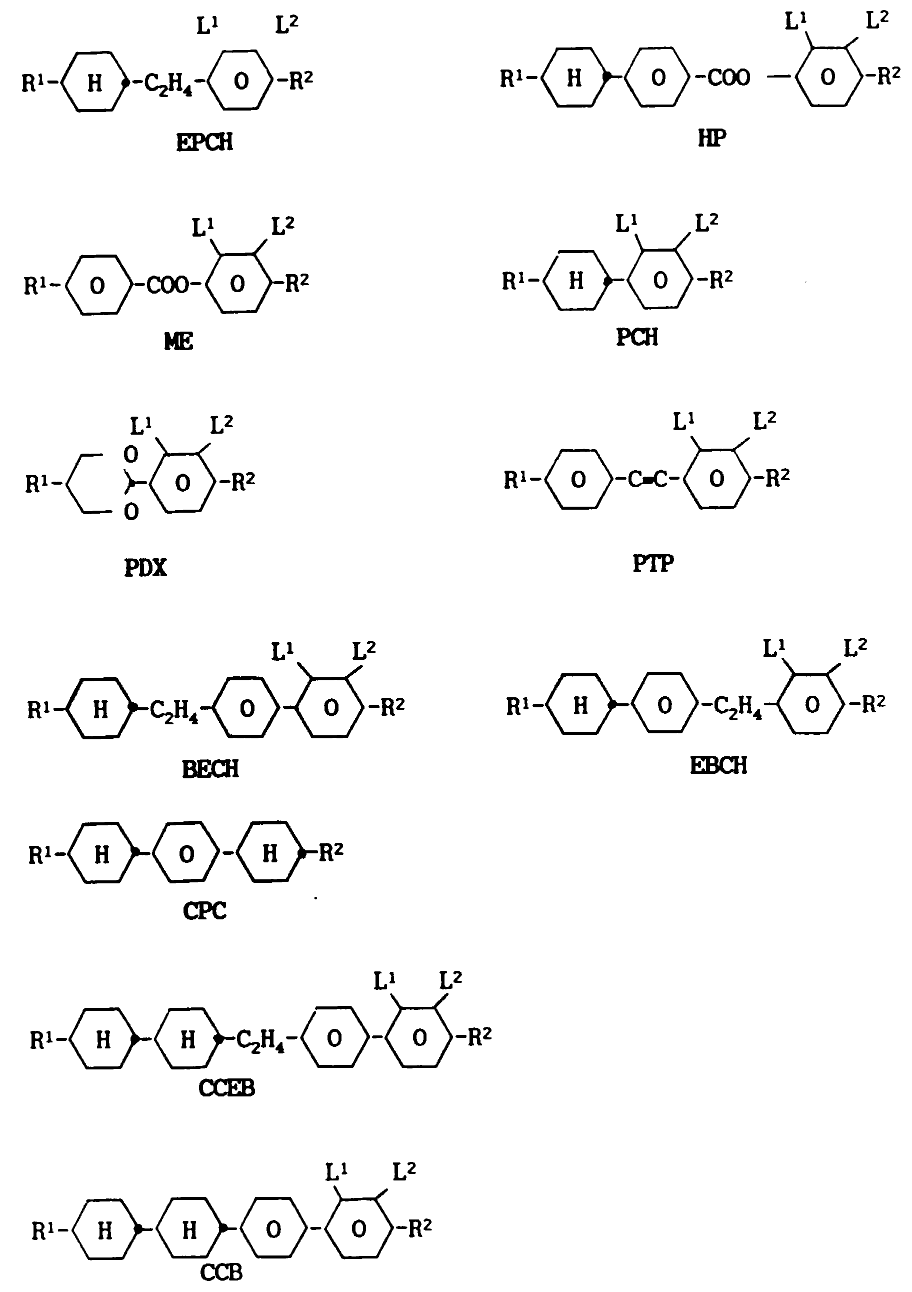

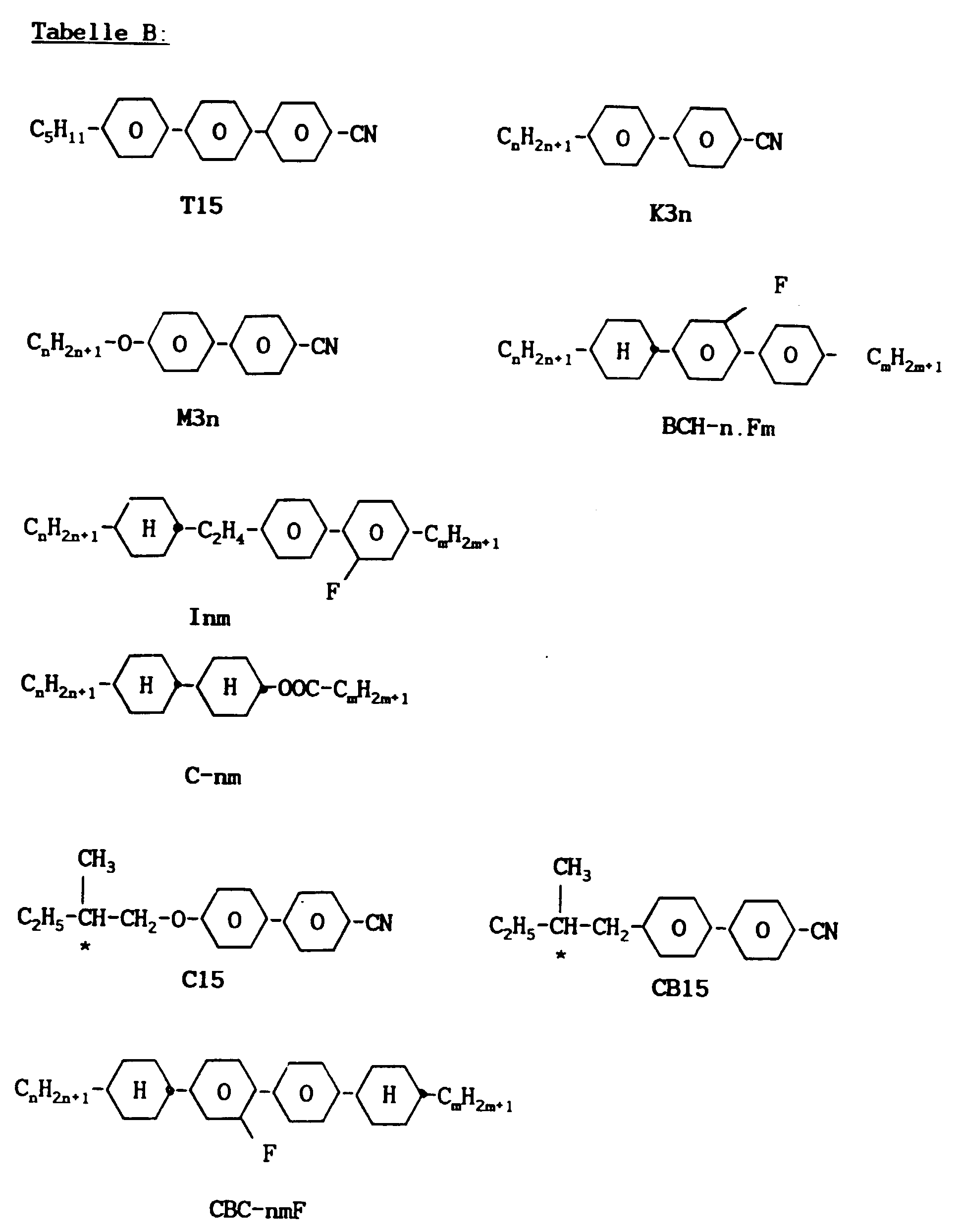

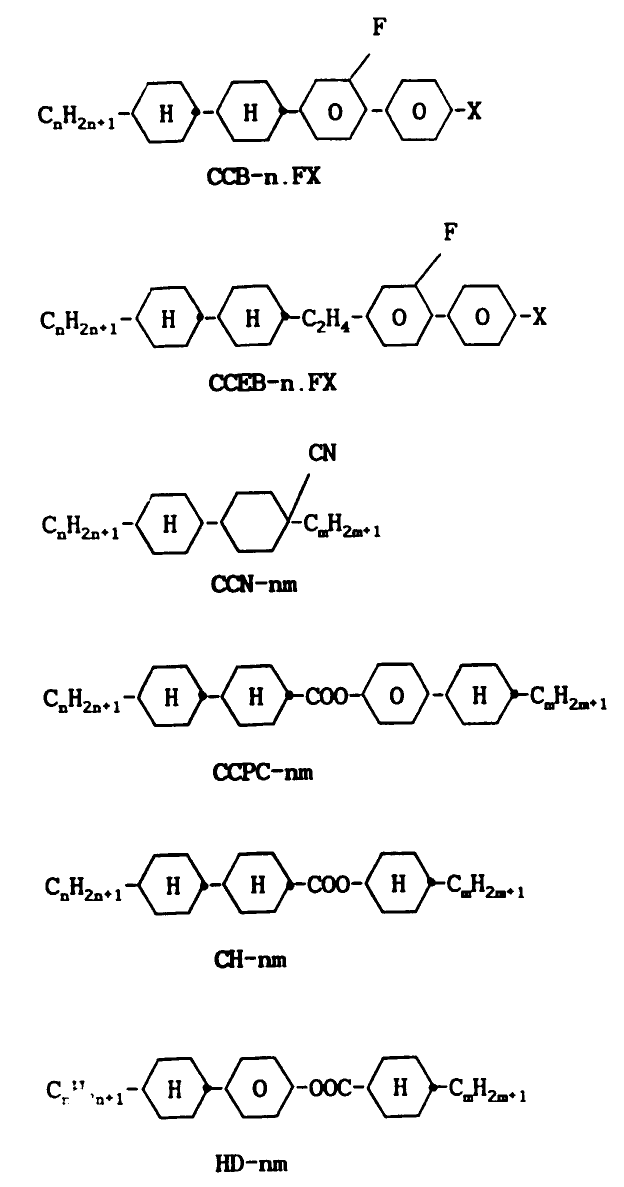

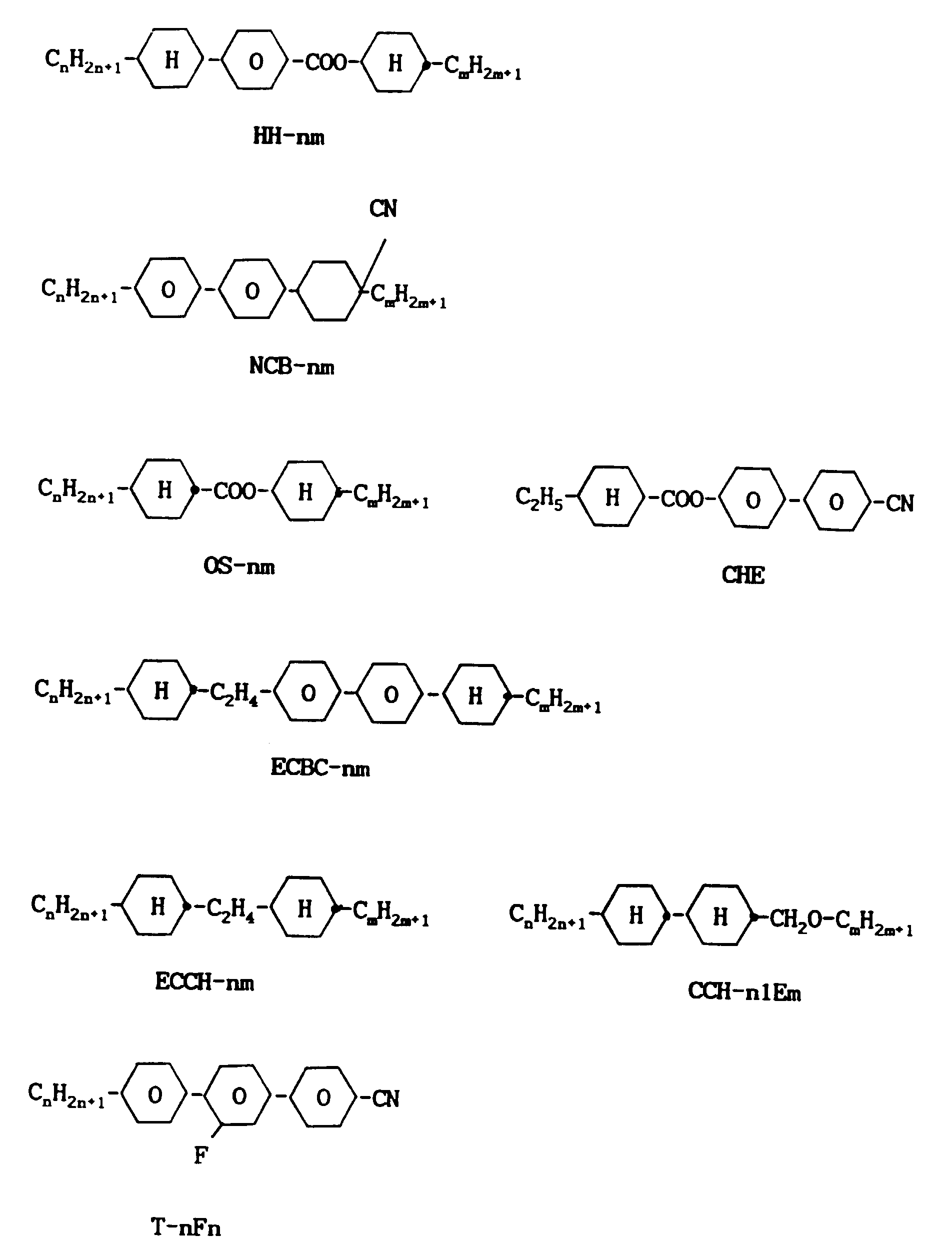

- DAST: Diethylaminoschwefeltrifluorid
- DCC: Dicyclohexylcarbodiimid
- DDQ: Dichlordicyanobenzochinon
- DIBALH: Diisobutylaluminiumhydrid
- DMSO: Dimethylsulfoxid
- KOT: Kalium-tertiär-butanolat
- THF: Tetrahydrofuran
- pTSOH: p-Toluolsulfonsäure

### Vergleichsbeispiel 1

Eine Lösung von 0,05 m 1-[trans-4-(trans-4-n-Propyl-cyclohexyl) -cyclohexyl]-2-(p-bromphenyl) -ethan (DE 3317597) und 0,05 m p-Trifluormethyl-phenylboronsäure (hergestellt aus p-Bromtrifluortoluol durch nacheinanderfolgende Umsetzung mit n-BuLi / B(OCH₃)₃ und H⁺/H₂O) in 100 ml Toluol und 50 ml Ethanol wird nach Zusatz von 0,0001 m Pd(Ph₃)₄ und 50 ml Na₂CO₃-Lösung (2M) 4 Stunden an Rückfluß gekocht. Nach üblicher extraktiver Aufarbeitung und nachfolgender Aufreinigung durch Chromatographie und Kristallisation erhält man 1-[trans-4-(trans-4-n-Propyl-cyclohexyl)-cyclohexyl]-2-(4-trifluromethylbiphenyl-4'-yl)-ethan.

### Beispiel 1 bis 48 :

Analog Vergleichsbeispiel 1 erhält man die folgenden Verbindungen der Teilformel Ia:

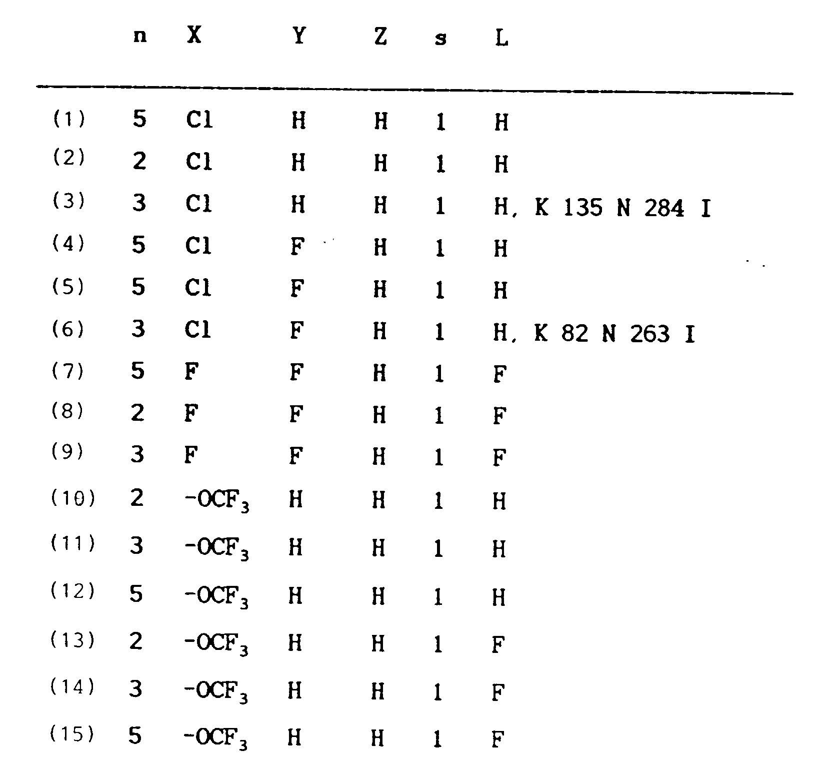

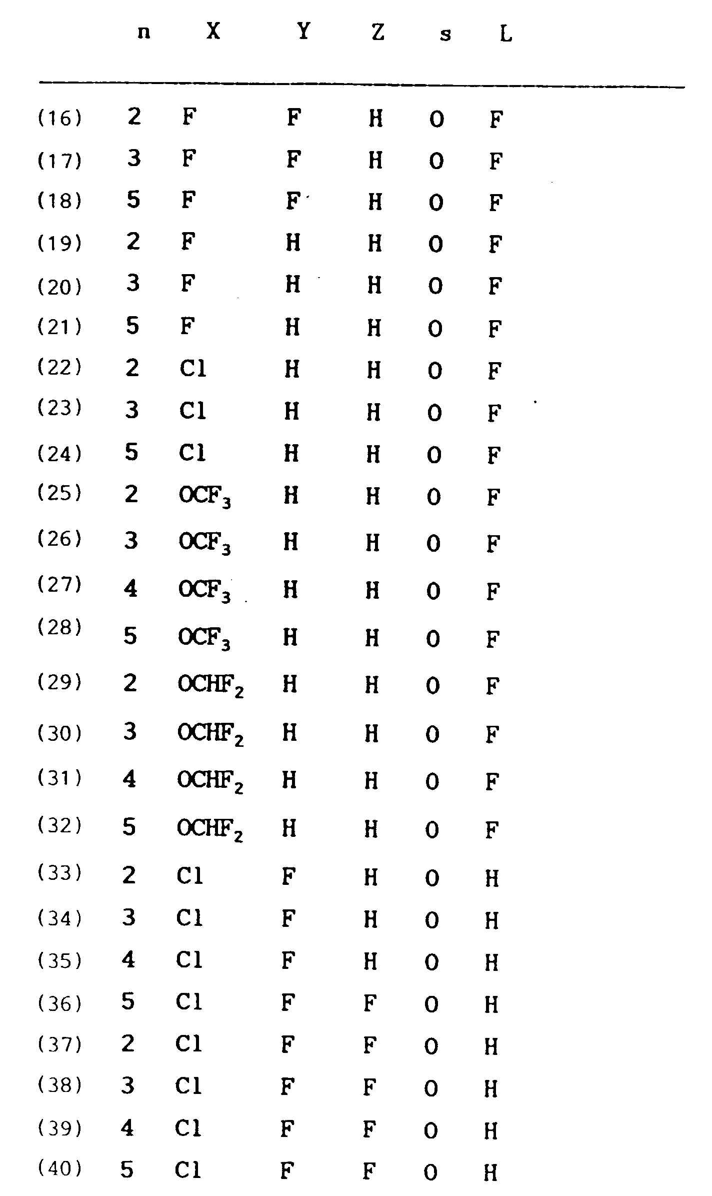

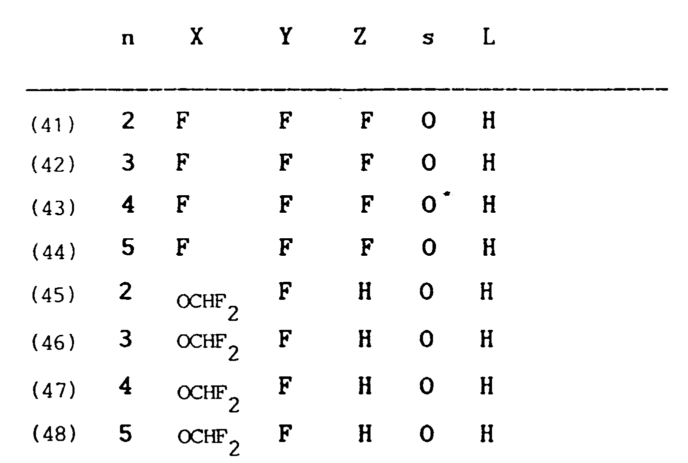

### Vergleichsbeispiel 2

a) Eine Lösung von 0,2 m 3,4-Difluorbrombenzol in 100 ml Diethylether wird bei -30° mit 0,2 m n-BuLi und anschließend mit einer Lösung von 0,2 m TiCl (OiPr)₃ in THF versetzt. Dann läßt man unter Rühren die Temperatur -10° erreichen, setzt 0,2 m 4-Oxocyclohexancarbonsäureethylester zu und rührt noch 2 Stunden bei Raumtemperatur. Nach extraktiver Aufarbeitung und Entfernen des Lösungsmittels nimmt man den Rückstand in 200 ml Toluol auf, gibt 1 g p-Toluolsulfonsäure zu und kocht 2 Stunden am Wasserabscheider. Nach extraktiver Aufarbeitung reinigt man das Produkt durch Chromatographie.
b) Das erhaltene Cyclohexenderivat wird in 100 ml THF gelöst und nach Zusatz von 5 g Pd-C bei Normaldruck hydriert. Nach Filtrieren, Eindampfen und basischer Etherspaltung erhält man nach Kristallisation trans-4-(3,4-Difluorphenyl)-Cyclohexancarbonsäure.
c) Die erhaltene Cyclohexancarbonsäure wird mit 50 ml Thionylchlorid über Nacht bei Raumtemperatur gerührt. Nach destillativer Reinigung wird das Säurechlorid durch Einleiten von Wasserstoff in Gegenwart von Pd-BaSO₄ nach Rosenmund in den Aldehyd übergeführt.
d) Eine Lösung von 0,1 m des erhaltenen Aldehyds und 0,1 m trans-4-(trans-4-n-Propyl-cyclohexyl)-cyclohexylmethylphosphoniumjodid in 100 ml THF wird unter Kühlen bei 0° mit KOT versetzt. Man rührt noch 4 Stunden bei Raumtemperatur nach und arbeitet nach Zusatz von 50 ml H₂O extraktiv auf.
e) Das Rohprodukt wird nach Zusatz von 5 g Pd-C in THF bei Normaldruck hydriert. Nach Filtrieren, Eindampfen und Reinigen durch Kristallisation erhält man 1-[trans-4-(trans-4-n-Propylcyclohexyl)-cyclohexyl]-2-[trans-4-(3,4-difluorphenyl)-cyclohexyl]-ethan.

### Beispiel bis

Analog Vergleichsbeispiel 2 erhält man die folgenden Verbindungen der Teilformel Ib:

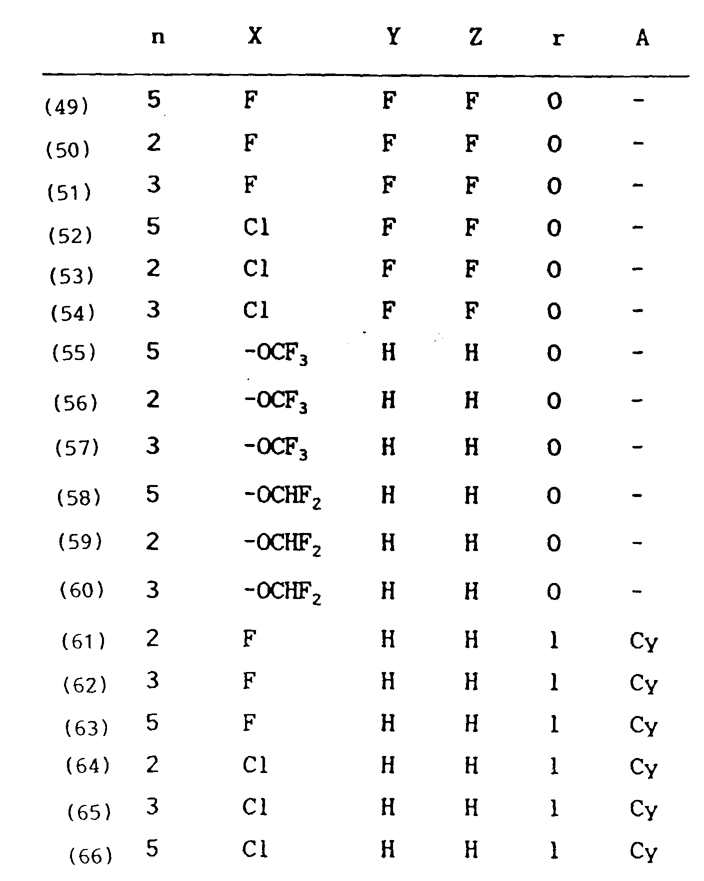

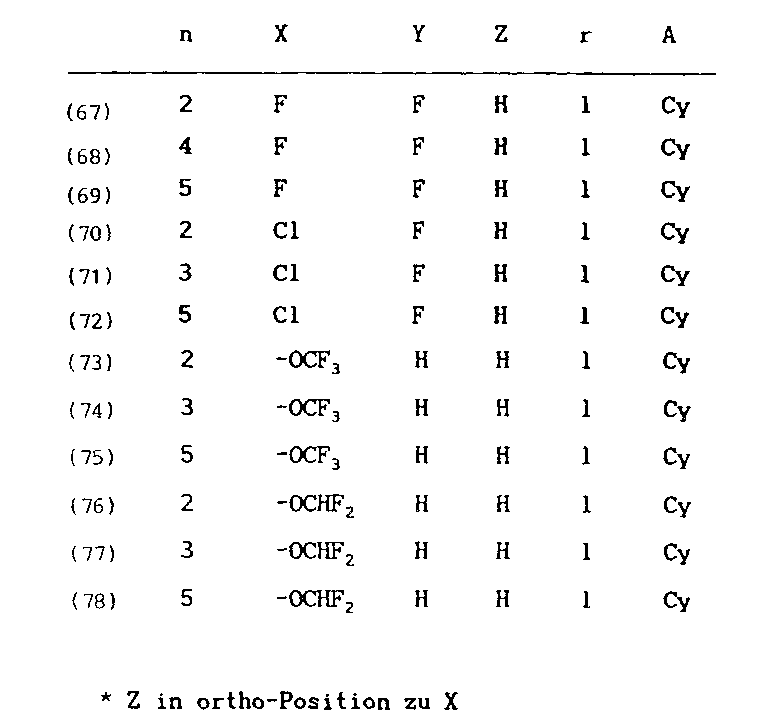

### Vergleichsbeispiel 3

Nach obigem Schema 12 werden 3 g 1-(trans,trans-4-n-Propylcyclohexylcyclohexyl)-2-(p-bromphenyl)-ethan mit 1,13 g p-Cyanophenylboronsäure und 0,01 g Tetrakistriphenylphosphinpalladium(0) in 20 ml Toluol mit 2 M Natriumcarbonatlösung (10 ml) und 4 ml Ethanol am Rückfluß bis zur Beendigung der Reaktion gekocht. Nach üblicher Aufarbeitung erhält man 1-(trans,trans-4-n-Propylcyclohexylcyclohexyl)-2-(4-cyan- biphenyl-4'-yl)-ethan, C 139 N > 300 I.

Analog erhält man die folgenden Verbindungen der Teilformel Iaa

| n | s | X |
|---|---|---|
| 2 | 1 | OCF₃ |
| 3 | 1 | OCF₃ |
| 5 | 1 | OCF₃ |
| 2 | 1 | OCHF₂ |
| 3 | 1 | OCHF₂ |
| 5 | 1 | OCHF₂ |

### Beispiel 79

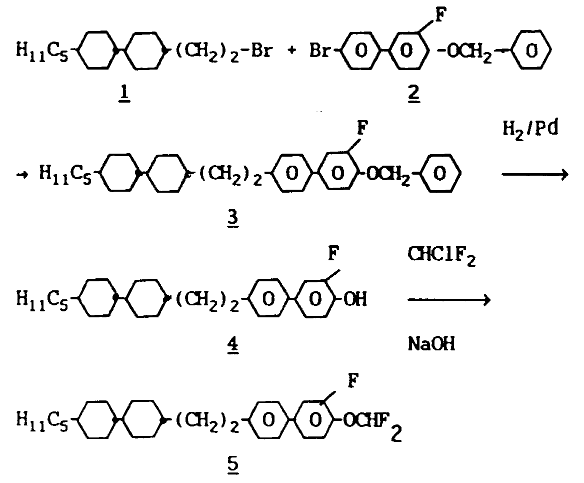

Gemäß obenstehendem Syntheseschema wird nach Überführung von 1 in die zinkorganische Verbindung durch eine Pd-(II)-katalysierte Kopplungsreaktion mit 2 die Verbindung 3 erhalten. Hydrogenolytische Spaltung des Benzylethers führt zum Phenol 4.

0,01 mol dieses Phenols werden in THF vorgelegt, 3,1 g 32%ige Natronlauge und 0,5 g Tetrabutylammoniumhydrogensulfat zugegeben, auf 50 °C erwärmt und unter Rühren solange Chloridfluormethan eingeleitet, bis dieses an einem mit Trockeneis gekühlten Kühler kondensiert. Nach dem Abkühlen wird die THF-Lösung von dem ausgefallenen schmierigen Produkt abdekantiert, eingeengt und das erhaltene 5 aus Ethanol umkristallisiert.

| Beispiel A | |
|---|---|
| PCH-5F | 8 % |
| PCH-6F | 6 % |
| PCH-7F | 5 % |
| CCP-20CF₃ | 6 % |
| CCP-30CF₃ | 9 % |
| CCP-40CF₃ | 6 % |
| CCP-50CF₃ | 9 % |
| BCH-3F.F | 9 % |
| BCH-5F.F | 8 % |
| ECCP-30CF₃ | 4 % |
| ECCP-50CF₃ | 4 % |
| CBC-33F | 2 % |
| CBC-53F | 2 % |
| CBC-55F | 2 % |
| CCB-3.FOCF₃ | 10 % |
| CCB-3.FF | 10 % |
| Klp. 92 °C | |
| Vₜₕ 2,4 V | |

| Beispiel B | |
|---|---|
| PCH-5F | 8 % |
| PCH-6F | 6 % |
| PCH-7F | 5 % |
| CCP-20CF₃ | 6 % |
| CCP-30CF₃ | 9 % |
| CCP-40CF₃ | 6 % |
| CCP-50CF₃ | 9 % |
| BCH-3F.F | 9 % |
| BCH-5F.F | 8 % |
| ECCP-30CF₃ | 4 % |
| ECCP-50CF₃ | 4 % |
| CBC-33F | 2 % |
| CBC-53F | 2 % |
| CBC-55F | 2 % |
| CCEB-3Cl.F | 10 % |
| CCB-3Cl.F | 10 % |
| Klp. 88 °C | |
| Vₜₕ 2,2 V | |

## Patentansprüche

1. Halogenierte Benzolderivate der Formel I 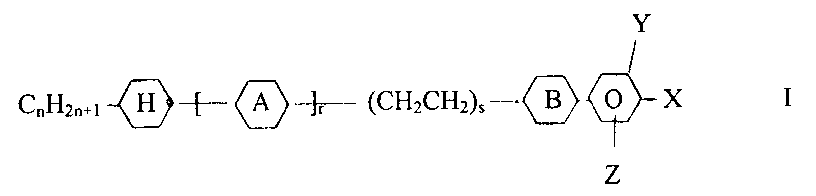 worin n 1 bis 10, die Ringe A und B jeweils trans-1,4-Cyclohexylen, oder Ring B auch 1,4-Phenylen oder 3-Fluor-1,4-phenylen, X F, Cl, -OCF₃ oder -OCHF₂, Y und Z jeweils unabhängig voneinander H oder F bedeuten, r = 1 oder im Falle s = 1, B = trans- 1,4-Cyclohexylen und gleichzeitig X = -OCF₃ oder -OCHF₂ auch r = 0, und s = 1 oder im Falle B = 1,4-Phenylen oder 3-Fluor-1,4-phenylen auch s = 0 bedeutet,
mit der Maßgabe, daß
im Falle r = 1, A = trans-1,4-Cyclohexylen und gleichzeitig X = F B 3-Fluor-1,4-phenylen oder trans-1,4-Cyclohexylen und/oder Y und Z jeweils Fluor bedeuten, wobei Verbindungen der Formel 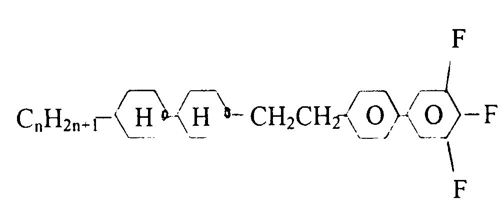 mit n = 1 - 10 ausgeschlossen sind.

2. Benzolderivate nach Anspruch 1, gekennzeichnet durch die Formel Ia 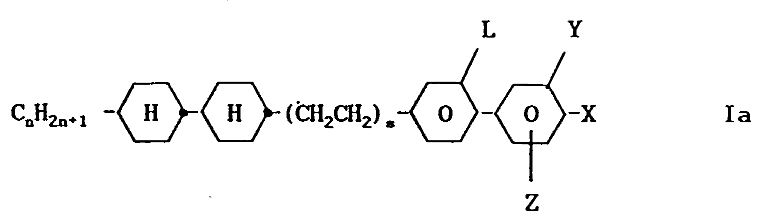 worin n 1 bis 7, s 0 oder 1, X F, Cl, -OCF₃ oder -OCHF₂ und Y, L und Z jeweils unabhängig voneinander H oder F bedeuten, mit der Maßgabe, daß im Falle X = F L = F, L = Y = F oder Y = Z = F.

3. Benzolderivate nach Anspruch 2, gekennzeichnet durch die Formel Iaa 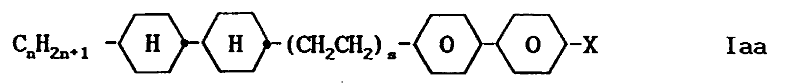 worin X Cl -OCF₃ oder -OCHF₂ ist.

4. Benzolderivate nach Anspruch 2, gekennzeichnet durch die Formel Iab 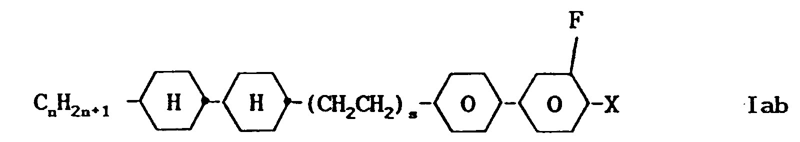 worin X Cl, -OCF₃ oder -OCHF₂ ist.

5. Benzolderivate nach Anspruch 2, gekennzeichent durch die Formel Iac 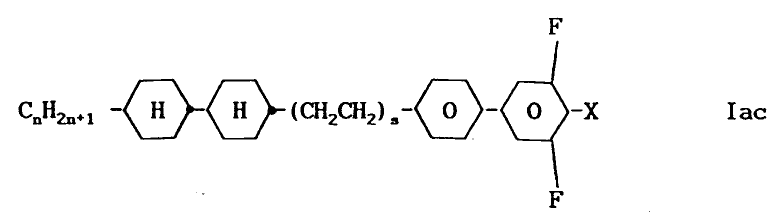 worin X F, Cl, -OCF₃ oder -OCHF₂ ist.

6. Benzolderivate nach Anspruch 2, gekennzeichnet durch die Formel Iad 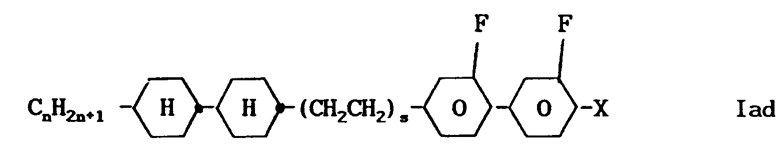 worin X F, Cl, -OCF₃ oder -OCHF₂ ist.

7. Benzolderivate nach Anspruch 2, gekennzeichnet durch die Formel Iae 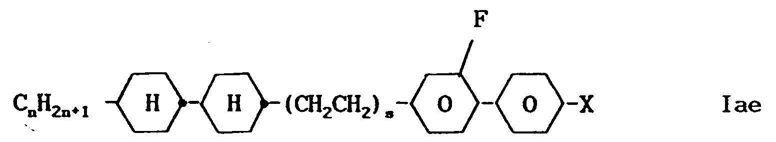 worin X F, Cl, -OCF₃ oder -OCHF₂ ist.

8. Halogenierte Benzolderivate nach Anspruch 1, gekennzeichnet durch die Formel Ib 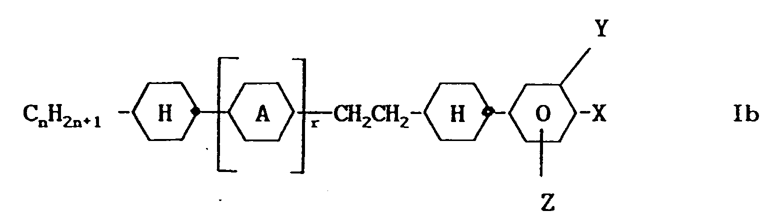 worin n 1 bis 7, r 0 oder 1, Ring A trans-1,4-Cyclohexylen, X F, Cl, -OCF₃ oder -OCHF₂ und Y und Z jeweils unabhängig voneinander H oder F bedeuten.

9. Benzolderivate nach Anspruch 8, gekennzeichnet durch die Formel Iba 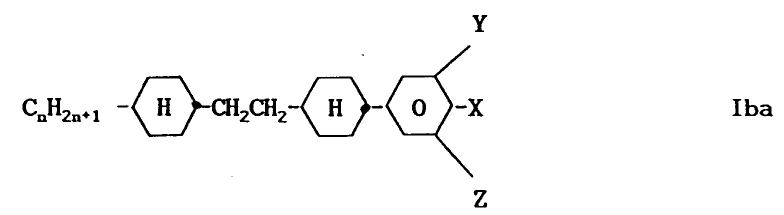 worin X -OCF₃ oder -OCHF₂ bedeutet und Y und Z jeweils unabhängig voneinander H oder F bedeuten.

10. Benzolderivate nach Anspruch 8, gekennzeichnet durch die Formel Ibd 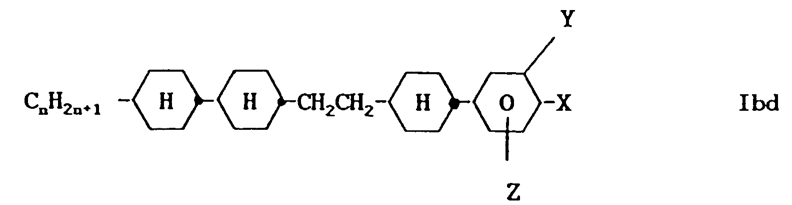 worin X, Y und Z die in Anspruch 8 angegebene Bedeutung haben.

11. Benzolderivate nach Anspruch 10, dadurch gekennzeichnet, daß X = F, Cl, -OCF₃ oder -OCHF₂ und Y = Z = H bedeutet.

12. Benzolderivate nach Anspruch 10, dadurch gekennzeichnet, daß X = F, Cl, -OCF₃ oder -OCHF_{2.} Y = F und Z = H bedeutet.

13. Benzolderivate nach Anspruch 13, dadurch gekennzeichnet, daß X = F oder Cl bedeutet.

14. Verwendung der Benzolderivate der Formel I nach Anspruch 1 als Komponenten flüssigkristalliner Medien für elektrooptische Anzeigen.

15. Flüssigkristallines Medium für elektrooptische Anzeigen mit mindestens zwei flüssigkristallinen Komponenten, dadurch gekennzeichnet, daß mindestens eine Komponente ein Benzolderivat der Formel I nach Anspruch 1 ist.

16. Flüssigkristallines Medium für elektrooptische Anzeigen mit einen oder mehreren halogenierten Benzolderivaten der Formel I 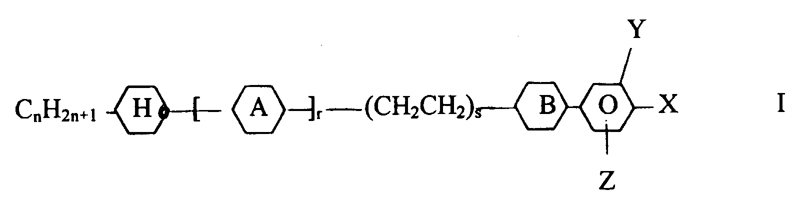 worin n 1 bis 10, die Ringe A und B jeweils trans-1,4-Cyclohexylen, oder Ring B auch 1,4-Phenylen oder 3-Fluor-1,4-phenylen, X F, Cl, -OCF₃ oder -OCHF₂, Y und Z jeweils unabhängig voneinander H oder F bedeuten, r = 1 oder im Falle s = 1, B = trans-1,4-Cyclohexylen und gleichzeitig X = -OCF₃ oder -OCHF₂ auch r = 0, und s = 1 oder im Falle B = 1,4-Phenylen oder 3-Fluor- 1,4-phenylen auch s = 0 bedeutet,
oder der folgenden Formel 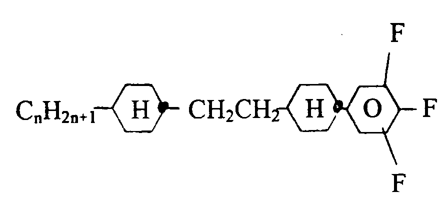 (n = 2, 3, 5)
dadurch gekennzeichnet, daß es als weiteren Bestandteil eine Verbindung der Formel 1 enthält
R'-L-E-R" 1
worin L und E, die gleich oder verscheiden sein können, jeweils unabhängig voneinander einen bivalenten Rest aus der aus -Phe-, -Phe-Phe-, -Phe-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc-trans-1,4-Cyclohexylen oder 1,4-Cyclo-hexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten, R' und R" entweder
jeweils unabhängig voneinander Alkyl, Alkenyl, Alkoxy, Alkoxyalkyl, Alkenyloxy oder Alkanoyloxy mit bis zu 8 Kohlenstoffatomen, oder
R" -F, -Cl, -NCS oder -(O)ᵢCH₃₋₍ₖ₊₁₎FₖClₗ, wobei i 0 oder 1 und k + 1 1, 2 oder 3 sind und R' Alkyl, Alkenyl, Alkoxy oder Alkoxyalkyl mit bis zu 8 Kohlenstoffatomen, oder
R" -CN und R' Alkyl, Alkoxy oder Alkenyl mit bis zu 8 Kohlenstoffatomen bedeutet.

17. Elektrooptische Anzeige auf der Basis einer Flüssigklistallanzeige, dadurch gekennzeichnet, daß die Flüssigkristallzelle ein Medium nach Anspruch 15 und 16 enthält.

## Claims

1. Halogenated benzene derivatives of the formula I 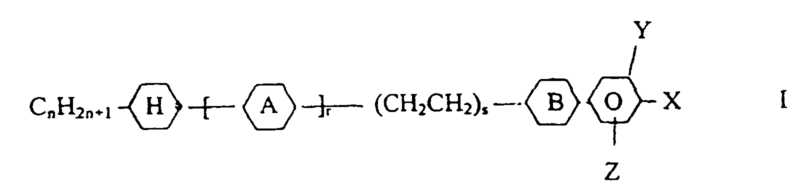 in which n is from 1 to 10, the rings A and B are each trans-1,4-cyclohexylene, or the ring B is alternatively 1,4-phenylene or 3-fluoro-1,4-phenylene, X is F, Cl, -OCF₃ or -OCHF₂, Y and Z are each, independently of one another, H or F, r = 1 or, in the case where s = 1, B = trans-1,4-cyclohexylene and simultaneously X = -OCF₃ or -OCHF₂, r is alternatively 0, and s = 1 or, in the case where B = 1,4-phenylene or 3-fluoro-1,4-phenylene, s is alternatively 0,
with the proviso that
in the case where r = 1, A = trans-1,4-cyclohexylene and simultaneously X = F, B is 3-fluoro-1,4-phenylene or trans-1,4-cyclohexylene and/or Y and Z are each fluorine, with the exception of compounds of the formula 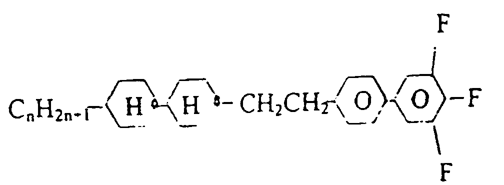 where n = 1-10.

2. Benzene derivatives according to Claim 1, characterized by the formula Ia 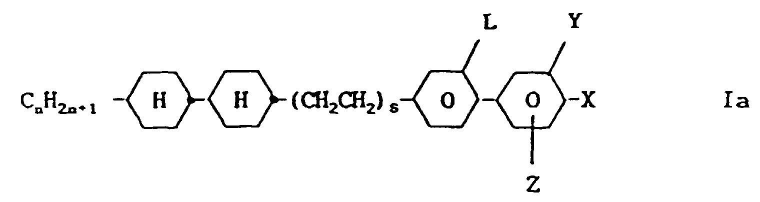 in which n is from 1 to 7, s is 0 or 1, X is F, Cl, -OCF₃ or -OCHF₂, and Y, L and Z are each, independently of one another, H or F, with the proviso that, in the case where X = F, L = F, L = Y = F or Y = Z = F.

3. Benzene derivatives according to Claim 2, characterized by the formula Iaa 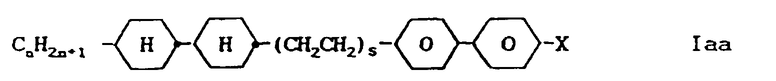 in which X is Cl, -OCF₃ or -OCHF₂.

4. Benzene derivatives according to Claim 2, characterized by the formula Iab 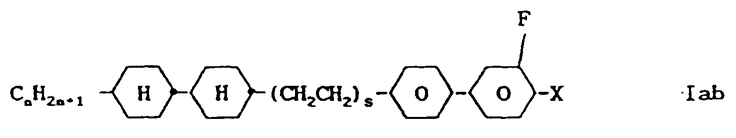 in which X is Cl, -OCF₃ or -OCHF₂.

5. Benzene derivatives according to Claim 2, characterized by the formula Iac 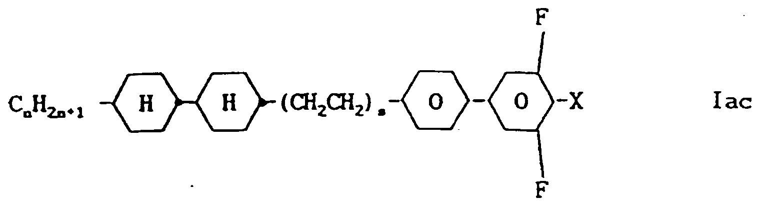 in which X is F, Cl, -OCF₃ or -OCHF₂.

6. Benzene derivatives according to Claim 2, characterized by the formula Iad 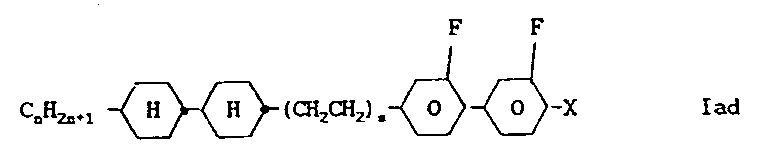 in which X is F, Cl, -OCF₃ or -OCHF₂.

7. Benzene derivatives according to Claim 2, characterized by the formula Iae 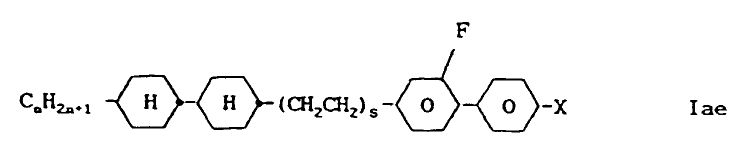 in which X is F, Cl, -OCF₃ or -OCHF₂.

8. Halogenated benzene derivatives according to Claim 1, characterized by the formula Ib 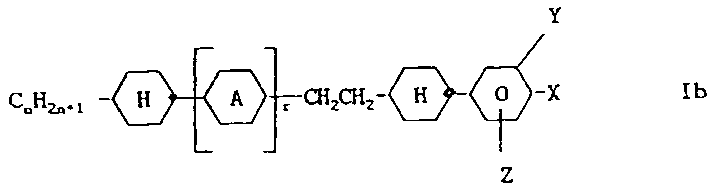 in which n is from 1 to 7, r is 0 or 1, the ring A is trans-1,4-cyclohexylene, X is F, Cl, -OCF₃ or -OCHF₂, and Y and Z are each, independently of one another, H or F.

9. Benzene derivatives according to Claim 8, characterized by the formula Iba 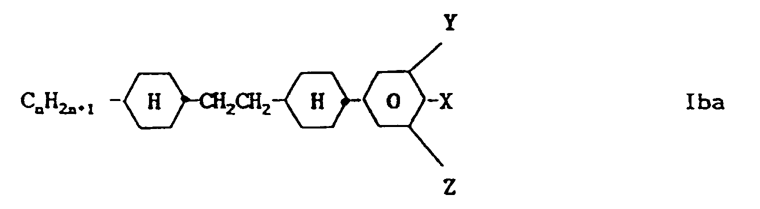 in which X is -OCF₃ or -OCHF₂, and Y and Z are each, independently of one another, H or F.

10. Benzene derivatives according to Claim 8, characterized by the formula Ibd 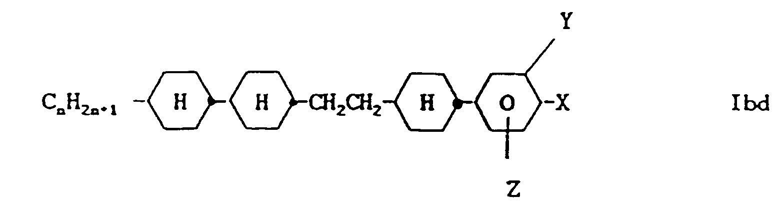 in which X, Y and Z are as defined in Claim 8.

11. Benzene derivatives according to Claim 10, characterized in that X = F, Cl, -OCF₃ or -OCHF₂, and Y = Z = H.

12. Benzene derivatives according to Claim 10, characterized in that X = F, Cl, -OCF₃ or -OCHF₂, Y = F and Z = H.

13. Benzene derivatives according to Claim 13, characterized in that X = F or Cl.

14. Use of benzene derivatives of the formula I according to Claim 1 as components of liquid-crystalline media for electro-optical displays.

15. Liquid-crystalline medium for electro-optical displays, having at least two liquid-crystalline components, characterized in that at least one component is a benzene derivative of formula I according to Claim 1.

16. Liquid-crystalline medium for electro-optical displays, containing one or more halogenated benzene derivatives of the formula I 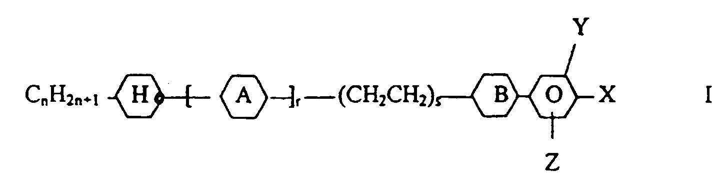 in which n is from 1 to 10, the rings A and B are each trans-1,4-cyclohexylene, or ring B is alternatively 1,4-phenylene or 3-fluoro-1,4-phenylene, X is F, Cl, -OCF₃ or -OCHF₂, Y and Z are each, independently of one another, H or F, r = 1 or, in the case where s = 1, B = trans-1,4-cyclohexylene and simultaneously X = -OCF₃ or -OCHF₂, r is alternatively 0, and s = 1 or, in the case where B = 1,4-phenylene or 3-fluoro-1,4-phenylene, s is alternatively 0,
or of the following formula 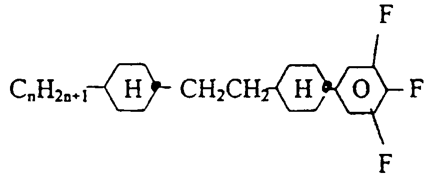 (n = 2, 3 or 5)
characterized in that it contains, as further constituents, a compound of the formula I.
R'-L-E-R" I
in which L and E, which may be identical or different, are each, independently of one another, a bivalent radical from the group consisting of -Phe-, -Phe-Phe-, -Phe-Cyc-, -Pyr-, -Dio-, -G-Phe- and -G-Cyc-, and mirror images thereof, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl, R' and R" are either
each, independently of one another, alkyl, alkenyl, alkoxy, alkoxyalkyl, alkenyloxy or alkanoyloxy having up to 8 carbon atoms, or
R" is -F, -Cl, -NCS or -(O)ᵢCH₃₋₍ₖ₊₁₎FₖClₗ, where i is 0 or 1 and k + 1 are 1, 2 or 3, and R' is alkyl, alkenyl, alkoxy or alkoxyalkyl having up to 8 carbon atoms, or
R" is -CN and R' is alkyl, alkoxy or alkenyl having up to 8 carbon atoms.

17. Electro-optical display based on a liquid-crystal display, characterized in that the liquid-crystal cell contains a medium according to Claim 15 or 16.

## Revendications

1. Dérivés benzéniques halogénés de formule I 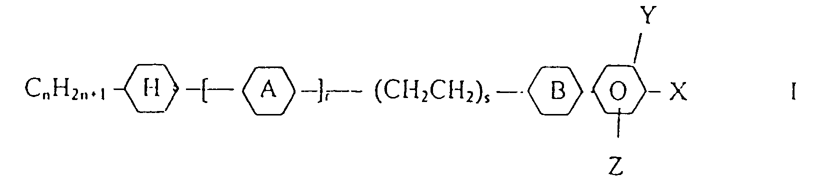 dans laquelle n va de 1 à 10, les cycles A et B représentent chacun le groupe *trans*-1,4-cyclohexylène, ou le cycle B représente également le groupe 1,4-phénylène ou 3-fluoro-1,4-phénylène, X représente F, Cl, -OCF₃ ou -OCHF₂, Y et Z représentent chacun, indépendamment l'un de l'autre, H ou F, r = 1, ou, dans le cas où s = 1, B représente le groupe trans-1,4-cyclohexylène et en même temps X = -OCF₃ ou -OCHF₂, r est aussi égal à 0, et s = 1 ou, dans le cas où B représente le groupe 1,4-phénylène ou 3-fluoro-1,4-phénylène, s est aussi égal à 0,
étant entendu que
lorsque r = 1, A représente le groupe *trans*-1,4-cyclohexylène et en même temps X = F, B représente le groupe 3-fluoro-1,4-phénylène ou trans-1,4-cyclohexylène et/ou Y et Z représentent chacun un atome de fluor,
les composés de formule 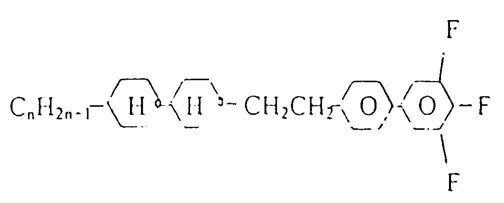 dans laquelle n = 1-10 étant exclus.

2. Dérivés benzéniques selon la revendication 1, caractérisés par la formule Ia 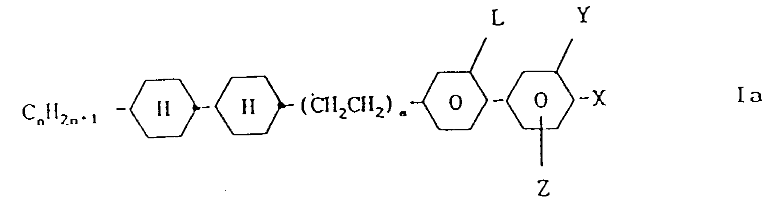 dans laquelle n va de 1 à 7, s est 0 ou 1, X représente F, Cl, -OCF₃ ou -OCHF₂, et Y, L et Z représentent chacun, indépendamment les uns des autres, H ou F, étant entendu que dans le cas où X = F L = F, L = Y = F ou Y = Z = F.

3. Dérivés benzéniques selon la revendication 2, caractérisés par la formule Iaa 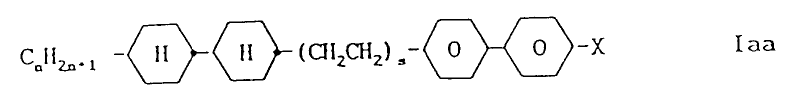 dans laquelle X est Cl, -OCF₃ ou -OCHF₂.

4. Dérivés benzéniques selon la revendication 2, caractérisés par la formule Iab 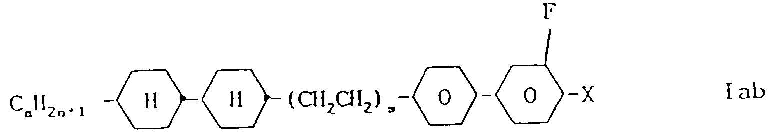 dans laquelle X est Cl, -OCF₃ ou -OCHF₂.

5. Dérivés benzéniques selon la revendication 2, caractérisés par la formule Iac 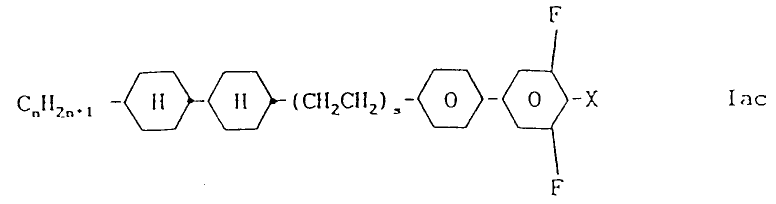 dans laquelle X est F, Cl, -OCF₃ ou -OCHF₂.

6. Dérivés benzéniques selon la revendication 2, caractérisés par la formule Iad 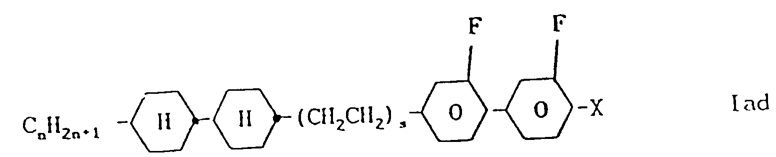 dans laquelle X est F, Cl, -OCF₃ ou -OCHF₂.

7. Dérivés benzéniques selon la revendication 2, caractérisés par la formule Iae 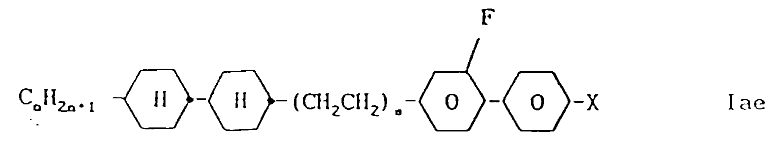 dans laquelle X est F, Cl, -OCF₃ ou -OCHF₂.

8. Dérivés benzéniques halogénés selon la revendication 1, caractérisés par la formule Ib 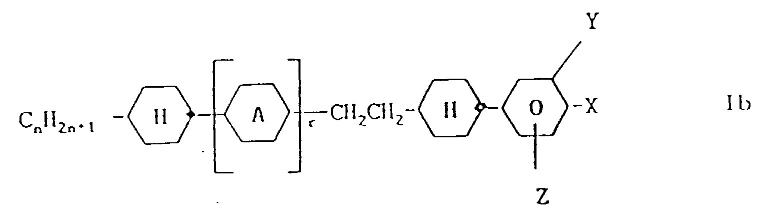 dans laquelle n va de 1 à 7, r est 0 ou 1, le cycle A représente le groupe *trans*-1,4-cyclohexylène, X représente F, Cl, -OCF₃ ou -OCHF₂, et Y et Z représentent chacun, indépendamment l'un de l'autre, H ou F.

9. Dérivés benzéniques selon la revendication 8, caractérisés par la formule Iba 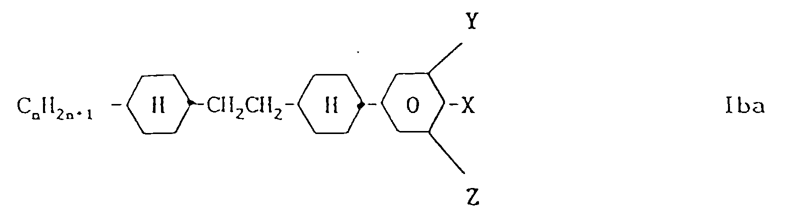 dans laquelle X représente -OCF₃ ou -OCHF₂, et Y et Z représentent chacun, indépendamment l'un de l'autre, H ou F.

10. Dérivés benzéniques selon la revendication 8, caractérisés par la formule Ibd 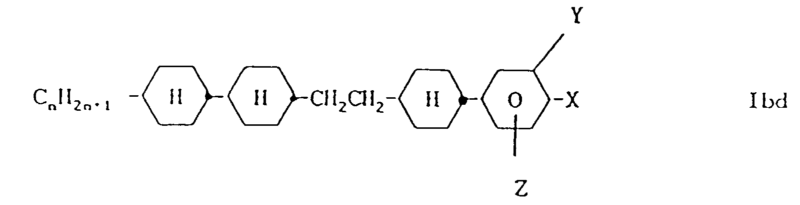 dans laquelle X, Y et Z ont les significations données dans la revendication 8.

11. Dérivés benzéniques selon la revendication 10, caractérisés en ce que X représente F, Cl, -OCF₃ ou -OCHF₂, et Y = Z = H.

12. Dérivés benzéniques selon la revendication 10, caractérisés en ce que X représente F, Cl, -OCF₃ ou -OCHF₂, Y = F et Z = H.

13. Dérivés benzéniques selon la revendication 12, caractérisés en ce que X représente F ou Cl.

14. Utilisation des dérivés benzéniques de formule I selon la revendication 1, en tant que composants de milieux à cristaux liquides pour afficheurs électro-optiques.

15. Milieu à cristaux liquides pour afficheurs électro-optiques à au moins deux composants de type cristal liquide, caractérisé en ce qu'au moins un composant est un dérivé benzénique de formule I selon la revendication 1.

16. Milieu à cristaux liquides pour afficheurs électro-optiques, comportant un ou plusieurs dérivés benzéniques halogénés de formule I 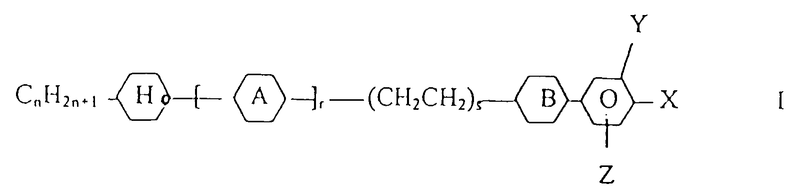 dans laquelle n va de 1 à 10, les cycles A et B représentent chacun le groupe trans-1,4-cyclohexylène, ou le cycle B représente également le groupe 1,4-phénylène ou 3-fluoro-1,4-phénylène, X représente F, Cl, -OCF₃ ou -OCHF₂, Y et Z représentent chacun, indépendamment l'un de l'autre, H ou F, r = 1, ou, dans le cas où s = 1, B représente le groupe *trans*-1,4-cyclohexylène et en même temps X = -OCF₃ ou -OCHF₂, r est aussi égal à 0, et s = 1 ou, dans le cas où B représente le groupe 1,4-phénylène ou 3-fluoro-1,4-phénylène, s est aussi égal à 0,
ou de formule suivante 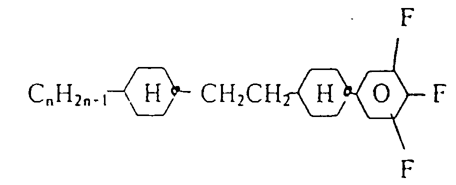 (n = 2, 3, 5)
caractérisé en ce qu'il contient, comme autre composant, un composé de formule 1
R'-L-E-R" 1
dans laquelle L et E, qui peuvent être identiques ou différents, représentent chacun, indépendamment l'un de l'autre, un radical bivalent choisi dans le groupe constitué par -Phe-, -Phe-Phe-, -Phe-Cyc-, -Pyr-, -Dio-, -G-Phe- et -G-Cyc- ainsi que leurs images dans un miroir, Phe signifiant un radical 1,4-phénylène non substitué ou substitué par le fluor, Cyc signifiant le radical *trans*-1,4-cyclohexylène ou 1,4-cyclohexénylène, Pyr signifiant le radical pyrimidine-2,5-diyle ou pyridine-2,5-diyle, Dio signifiant le radical 1,3-dioxanne-2,5-diyle et G signifiant le radical 2-(*trans*-1,4-cyclohexyl)éthyle, pyrimidine-2,5-diyle, pyridine-2,5-diyle ou 1,3-dioxanne-2,5-diyle,
R' et R" soit
représentent chacun indépendamment l'un de l'autre un groupe alkyle, alcényle, alcoxy, alcoxyalkyle, alcényloxy ou alcanoyloxy ayant jusqu'à 8 atomes de carbone, soit
R" représente -F, Cl, -NCS ou -(O)ᵢCH₃₋₍ₖ₊₁₎FₖCl₁, ᵢ étant égal à 0 ou 1, et _{k + 1} valant 1, 2 ou 3, et R' étant un groupe alkyle, alcényle, alcoxy ou alcoxyalkyle ayant jusqu'à 8 atomes de carbone, ou
R" représente -CN et R' représente un groupe alkyle, alcoxy ou alcényle ayant jusqu'à 8 atomes de carbone.

17. Afficheur électro-optique à base d'une cellule à cristaux liquides, caractérisé en ce que la cellule à cristaux liquides contient un milieu selon la revendication 15 ou 16.
